# EUROPEAN PATENT APPLICATION

(11) **EP 1 911 762 A1**
(43) Date of publication of application: **16.04.2008**
(21) Application number: 06121768.3
(22) Date of filing: 04.10.2006
(51) Int. Cl.: C07K 5/06, C07D 209/04, C07D 209/08, C07D 209/12, C07D 213/69, C07D 215/38, C07D 231/56, C07D 295/08, A61K 31/00, A61K 31/404, A61K 31/416, A61K 31/4412, A61K 31/4453, A61P 9/12

(54) **Amino alcohols and their use as renin inhibitors**

(71) Applicant: Speedel Experimenta AG, 4123 Allschwil (CH)
(72) Inventor: Herold, Peter, 4123 Allschwil (CH); Mah, Robert, 4123 Allschwil (CH); Marti, Christiane, 4123 Allschwil (CH)
(74) Representative: Maué, Paul Georg

(57) **Abstract**

The invention provides substituted amino alcohols of the general formula (I) 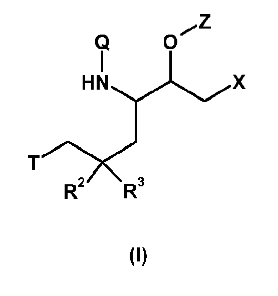 and the salts thereof, preferably the pharmaceutically acceptable salts thereof;
where R², R³, Q, T, X and Z have the definitions elucidated in more detail in the description, to a process for their preparation and to the use of these compounds as medicines, in particular as renin inhibitors. Moreover, the enzymatic substrate portion of the compound is simultaneously a substrate for a membrane transporter.

## Description

### Field of the Invention

The present invention relates to substituted amino alcohols, to processes for their preparation and to the use of the compounds as medicaments, in particular as renin inhibitors. Moreover, a process for enhancing the membrane transportation of such compounds is provided.

### Background of the Invention

It was long thought that intestinal absorption of most drugs proceeded by passive diffusion, in which the lipid solubility of the drug molecule was the determining factor. However, many water-soluble compounds have been shown to move well across cell membranes utilizing specialized carrier-mediated transport mechanisms. These membrane transporters play a key role in determining exposure of cells or organisms to a variety of solutes including nutrients and cellular by-products, as well as drug molecules. Efforts have been made to improve drug bioavailability by using different pro-moieties targeting various active transportation systems present in the small intestine. Examples of transportation systems include peptide transporters, organic cation transporters, organic anion transporters, glucose transporters, vitamin transporters, bile acid transporters, fatty acid transporters, phosphate transporters, monocarboxylic acid transporters, bicarbonate transporters, ABC transporters, nucleoside transporters and amino acid transporters, as described by H.-C. Shi et al. in: R. Mannhold, H. Kubinyi, G. Folkers, Eds., Methods and Principles in Medicinal Chemistry, Wiley-VCH, Weinheim, 2003, pp. 245-287, herein incorporated by reference. All of these transporters are mainly located in the brush border membrane with variable distribution along the gastrointestinal tract, and show diverse substrate specificities.
The peptide transporter-1 (PEPT1) is known to play a critical role in the absorption of diverse drugs and prodrugs from the intestinal tract. PEPT1 is located in the apical enterocytic membrane of the upper small intestine where it serves as a symporter, using an electrochemical proton gradient as its driving force. Besides the intestinal tract, PEPT1 is also expressed in the pancreas, bile ducts and kidneys, where it may play a role in the re-uptake of filtered peptides. Human PEPT1 (hPEPT1) contains 708 amino acids oriented in 12 membrane-spanning domains.

Many pharmaceuticals are known to utilize the intestinal PEPT1 to gain entry into the systematic circulation. Such pharmaceuticals include β-lactam antibiotics such as penicillins and cephalosporins, ACE-inhibitors, second generation peptidic renin inhibitors, thrombin inhibitors and the dipeptide-like anti-neoplastic drug bestatin, as well as prodrugs of ganciclovir, L-Dopa and pamidronate.
Renin (EC 3.4.99.19) is a member of the well-studied family of aspartic proteinases. It controls the first and rate-limiting step of the renin-angiotensin system catalyzing the cleavage of the Leu10-Val11 peptide bond of angiotensinogen to release the decapeptide angiotensin I. A large variety of peptidic inhibitors of human renin with different transition-state analogs of the scissile peptide bond have been developed in the past 3 decades. As none of these compounds has survived all stages of drug development, newer classes (third generation) of non-peptide renin inhibitors have recently been reported, for example, in EP 678503. However, with regard especially to renin inhibition, there is still a need for highly potent active ingredients. In this context, the improvement of the pharmacokinetic properties is at the forefront. These properties directed to better bioavailability are, for example, absorption, metabolic stability, solubility or lipophilicity.
We have found unexpectedly that certain amino acid derivatives of third generation, non-peptidic amino alcohol-based renin inhibitors have increased bioavailabilty, in particular oral bioavailability. These derivatives are comprised of a chemical moiety attached to substituted amino alcohols, whereby the chemical moiety promotes uptake of the compound by a gastrointestinal tract membrane transporter, or other membrane transporter, thereby enhancing oral bioavailability. Examples of membrane transporters include peptide transporters, organic cation transporters, organic anion transporters, glucose transporters, vitamin transporters, bile acid transporters, fatty acid transporters, phosphate transporters, monocarboxylic acid transporters, bicarbonate transporters, ABC transporters, nucleoside transporters and amino acid transporters. Some of the third generation, non-peptidic amino alcohol-based renin inhibitors have been disclosed in WO2005/090305.

### Detailed Description of the Invention

The invention therefore provides a process for enhancing the membrane transportation of substituted amino alcohols of the general formula (I) 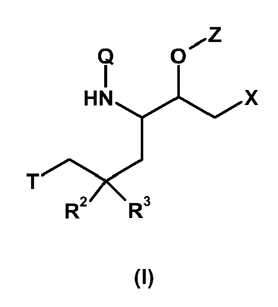 and the salts thereof, preferably the pharmaceutically acceptable salts thereof; where R¹ is aryl or a nitrogen-containing heterocyclyl, especially benzoimidazolyl, benzooxazolyl, benzothiazolyl, dihydro-2H-benzo[1,4]oxazinyl, dihydro-3H-benzo[1,4]oxazinyl, dihydro-2H-benzo[1,4]thiazinyl, dihydro-1H-pyrido[2,3-b][1,4]oxazinyl, imidazo[1,2-a]pyridyl, imidazo[1,5-a]pyridyl, indazolyl, indolyl, isoquinolyl, [1,5]naphthyridyl, phenyl, phthalazinyl, pyridyl, pyrimidinyl, 1H-pyrrolo[2,3-b]pyridyl, 1H-pyrrolo[2,3-c]pyridyl, 1H-pyrrolo[3,2-b]pyridyl, quinazolinyl, quinolyl, quinoxalinyl, tetrahydroimidazo[1,2-a]pyridyl, tetrahydroimidazo[1,5-a]pyridyl, tetrahydroisoquinolyl, tetrahydroquinolyl, tetrahydroquinoxalinyl, [1,2,3]triazolo[1,5-a]pyridyl or [1,2,4]triazolo[4,3-a]pyridyl, each of which is substituted by 1-4 acyl-C₁₋₈-alkoxy-C₁₋₈-alkoxy, acyl-C₁₋₈-alkoxy-C₁₋₈-alkyl, (N-acyl)-C₁₋₈-alkoxy-C₁₋₈-alkylamino, C₁₋₈-alkanoyl, C₁₋₈-alkoxy, C₁₋₈-alkoxy-C₁₋₈-alkanoyl, C₁₋₈-alkoxy-C₁₋₈-alkoxy, C₁₋₈-alkoxy-C₁₋₈-alkoxy-C₁₋₈-alkyl, C₁₋₈-alkoxy-C₁₋₈-alkyl, (N-C₁₋₈-alkoxy)-C₁₋₈-alkylaminocarbonyl-C₁₋₈-alkoxy, (N-C₁₋₈-alkoxy)-C₁₋₈-alkylaminocarbonyl-C₁₋₈-alkyl, C₁₋₈-alkoxy-C₁₋₈-alkyl-carbamoyl, C₁₋₈-alkoxy-C₁₋₈-alkyl-carbonylamino, C₁₋₈-alkoxy-C₁₋₈-alkyl-heterocydyl, C₁₋₈-alkoxy-carbonyl, C₁₋₈-alkoxy-carbonyl-C₁₋₈-alkoxy, C₁₋₈-alkoxycarbonyl-C₁₋₈-alkyl, C₁₋₈-alkoxycarbonylamino, C₁₋₈-alkoxy-carbonylamino-C₂₋₈-alkoxy, C₁₋₈-alkoxycarbonylamino-C₁₋₈-alkyl, C₁₋₈-alkyl, (N-C₁₋₈-alkyl)-C₁₋₈-alkoxy-C₁₋₈-alkyl-carbamoyl, (N-C₁₋₈-alkyl)-C₁₋₈-alkoxy-C₁₋₈-alkylcarbonylamino, (N-C₁₋₈-alkyl)-C₁₋₈-alkoxy-carbonylamino, (N-C₁₋₈-alkyl)-C₁₋₈-alkylcarbonylamino-C₂₋₈-alkoxy, (N-C₁₋₈-alkyl)-C₁₋₈-alkylcarbonylamino-C₁₋₈-alkyl, (N-C₁₋₈-alkyl)-C₁₋₈-alkylsulfonylamino-C₂₋₈-alkoxy, (N-C₁₋₈-alkyl)-C₁₋₈-alkylsulfonylamino-C₁₋₈-alkyl, C₁₋₈-alkyl-amidinyl, C₁₋₈-alkylamino-C₂₋₈-alkoxy, di-C₁₋₈-alkylamino-C₂₋₈-alkoxy, C₁₋₈-alkylamino-C₁₋₈-alkyl, di-C₁₋₈-alkylamino-C₁₋₈-alkyl, C₁₋₈-alkylaminocarbonyl-C₁₋₈-alkoxy, di-C₁₋₈-alkylaminocarbonyl-C₁₋₈-alkoxy, C₁₋₈-alkylamino-carbonyl-C₁₋₈-alkoxy-C₁₋₈-alkyl, C₁₋₈-alkylaminocarbonyl-C₁₋₈-alkyl, di-C₁₋₈-alkylaminocarbonyl-C₁₋₈-alkyl, C₁₋₈-alkylaminocarbonylamino-C₂₋₈-alkoxy, C₁₋₈-alkylaminocarbonylamino-C₁₋₈-alkyl, C₁₋₈-alkylcarbonylamino, C₁₋₈-alkylcarbonylamino-C₂₋₈-alkoxy, C₁₋₈-alkylcarbonylamino-C₁₋₈-alkyl, C₁₋₈-alkylcarbonyloxy-C₂₋₈-alkoxy, C₁₋₈-alkylcarbonyloxy-C₁₋₈-alkyl, C₁₋₈-alkylsulfonyl, C₁₋₈-alkylsulfonyl-C₁₋₈-alkoxy, C₁₋₈-alkylsulfonyl-C₁₋₈-alkyl, C₁₋₈-alkylsulfonylamino-C₂₋₈-alkoxy, C₁₋₈-alkylsulfonylamino-C₁₋₈-alkyl, optionally N-mono- oder N,N-di-C₁₋₈-alkylated amino, aryl-C₀₋₈-alkoxy, aryl-C₀₋₈-alkyl, optionally N-mono- oder N,N-di-C₁₋₈-alkylated carbamoyl-C₀₋₈-alkoxy, carboxy-C₁₋₈-alkoxy, carboxy-C₁₋₈-alkoxy-C₁₋₈-alkyl, carboxy-C₁₋₈-alkyl, C₁₋₈-alkyl, cyano-C₂₋₈-alkoxy, cyano-C₁₋₈-alkyl, C₃₋₈-cycloalkyl-C₁₋₈-alkoxy, C₃₋₈-cycloalkyl-carbamoyl-C₀₋₈-alkyl, C₃₋₈-cycloalkylcarbonylamino-C₂₋₈-alkoxy, C₃₋₈-cycloalkylcarbonylamino-C₁₋₈-alkyl, O,N-dimethylhydroxylamino-C₁₋₈-alkyl, halogen, halogen-C₁₋₈-alkoxy, halogen-C₁₋₈-alkyl, heterocydyl-C₀₋₈-alkoxy, heterocydyl-C₀₋₈-alkyl, heterocyclyl-carbonyl, hydroxy-C₂₋₈-alkoxy-C₁₋₈-alkoxy, hydroxy-C₂₋₈-alkoxy-C₁₋₈-alkyl, hydroxy-C₁₋₈-alkyl, O-methyloximyl-C₁₋₈-alkyl, oxide or oxo;
R² und R³ are, independently from each other, hydrogen or C₁₋₈-alkyl or, together with the carbon atom they are linked to, are C₃₋₈-cycloalkyl;
R⁴ is C₁₋₈-alkyl, C₁₋₈-hydroxyalkyl, C₁₋₈-alkoxy-C₁₋₈-alkyl, C₁₋₈-alkanoyloxy-C₁₋₈-alkyl, C₁₋₈-aminoalkyl, C₁₋₈-alkylamino-C₁₋₈-alkyl, C₁₋₈-dialkylamino-C₁₋₈-alkyl, C₁₋₈-alkanoylamido-C₁₋₈-alkyl, HO(O)C-C₁₋₈-alkyl, C₁₋₈-alkyl-O-(O)C-C₁₋₈-alkyl, H₂N-C(O)-C₁₋₈-alkyl, C₁₋₈-alkyl-HN-C(O)-C₁₋₈-alkyl, (C₁₋₈-alkyl)₂N-C(O)-C₁₋₈-alkyl, C₂₋₈-alkenyl, C₂₋₈-alkynyl, cyano-C₁₋₈-alkyl, halo-C₁₋₈-alkyl, optionally substituted aryl-C₀₋₈-alkyl, optionally substituted C₃₋₈-cycloalkyl-C₀₋₈-alkyl or optionally substituted heterocyclyl-C₀₋₈-alkyl
whereby,
each of the residues, mentioned above for R⁴, bearing a hydroxy-group are optionally substituted at that hydroxy-group by a radical (A) whereby an ester bond is formed; ;
R⁵ is hydrogen or C₁₋₈-alkyl;
R⁶ is optionally carboxy- or hydroxy-substituted C₁₋₈-alkyl or optionally carboxy- or hydroxy-substituted aryl-C₀₋₈-alkyl;
R⁷ is C₁₋₈-alkyl;
R⁸ and R⁹, together with the nitrogen atom to which they are bonded, are a 5-7-membered ring;
(A) represents a mono- or di-peptidic residue of one or two of the 20 natural amino acids, said residue being formally obtained from the mono- or di-peptide by elimination of the OH-group from the C-terminal end;
Q is hydrogen or represents a radical (A) whereby an amide bond is formed or a group of the formula -C(O)OCHR⁶OC(O)R⁷;
T is R¹, R¹-C(O)- or R¹-C(O)-NR⁵- ;
X is -NR⁵-C(O)R⁴, -Alk-C(O)-NR⁵R⁴ or -NR⁸R⁹, where Alk represents C₁₋₈-alkylene; and
Z is hydrogen or represents a radical (A) whereby an ester bond is formed;
characterized in that
(i) a radical (A) is present in at least one of R⁴, Q or Z; or else
(ii) at least Q is a group of the formula -C(O)OCHR⁶OC(O)R⁷.

The invention further provides substituted amino alcohols of the general formula (I) 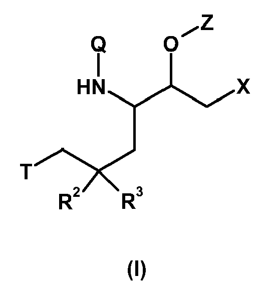 and the salts thereof, preferably the pharmaceutically acceptable salts thereof; where
A) X is -NR⁵-C(O)R⁴, -Alk-C(O)-NR⁵R⁴ or -NR⁸R⁹, where Alk represents C₁₋₈-alkylene; and
   T is R¹, R¹-C(O)- or R¹-C(O)-NR⁵-; and
   R¹ is a substituted nitrogen-containing heterocyclyl; or
B) X is -NR⁵-C(O)R⁴ or -NR⁸R⁹, where Alk represents C₁₋₈-alkylene; and
   T is R¹, R¹-C(O)- or R¹-C(O)-NR⁵-; and
   R¹ is substituted aryl; or
C) X is -Alk-C(O)-NR⁵R⁴ , where Alk represents C₁₋₈-alkylene and R⁴ is tetrahydropyranyl-C₀-C₄-alkyl; and
   T is R¹; and
   R¹ is substituted aryl;
R² und R³ are, independently from each other, hydrogen or C₁₋₈-alkyl or, together with the carbon atom they are linked to, are C₃₋₈-cycloalkyl;
R⁴ is C₁₋₈-alkyl, C₁₋₈-hydroxyalkyl, C₁₋₈-alkoxy-C₁₋₈-alkyl, C₁₋₈-alkanoyloxy-C₁₋₈-alkyl, C₁₋₈-aminoalkyl, C₁₋₈-alkylamino-C₁₋₈-alkyl, C₁₋₈-dialkylamino-C₁₋₈-alkyl, C₁₋₈-alkanoylamido-C₁₋₈-alkyl, HO(O)C-C₁₋₈-alkyl, C₁₋₈-alkyl-O-(O)C-C₁₋₈-alkyl, H₂N-C(O)-C₁₋₈-alkyl, C₁₋₈-alkyl-HN-C(O)-C₁₋₈-alkyl, (C₁₋₈-alkyl)₂N-C(O)-C₁₋₈-alkyl, C₂₋₈-alkenyl, C₂₋₈-alkynyl, cyano-C₁₋₈-alkyl, halo-C₁₋₈-alkyl, optionally substituted aryl-C₀₋₈-alkyl, optionally substituted C₃₋₈-cydoalkyl-C₀₋₈-alkyl or optionally substituted heterocyclyl-C₀₋₈-alkyl
whereby,
each of the residues, mentioned above for R⁴, bearing a hydroxy-group are optionally substituted at that hydroxy-group by a radical (A) whereby an ester bond is formed;
R⁵ is hydrogen or C₁₋₈-alkyl;
R⁶ is optionally carboxy- or hydroxy-substituted C₁₋₈-alkyl or optionally carboxy- or hydroxy-substituted aryl-C₀₋₈-alkyl;
R⁷ is C₁₋₈-alkyl;
R⁸ and R⁹, together with the nitrogen atom to which they are bonded, are a 5-7-membered ring;
(A) represents a mono- or di-peptidic residue of one or two of the 20 natural amino acids, said residue being formally obtained from the mono- or di-peptide by elimination of the OH-group from the C-terminal end;
Q is hydrogen or represents a radical (A) whereby an amide bond is formed or a group of the formula -C(O)OCHR⁶OC(O)R⁷;
Z is hydrogen or represents a radical (A) whereby an ester bond is formed; and
(i) a radical (A) is present in at least one of R⁴, Q or Z; or else
(ii) at least Q is a group of the formula -C(O)OCHR⁶OC(O)R⁷.

Examples of R¹ as substituted aryl or a substituted nitrogen-containing heterocyclyl, are especially benzoimidazolyl, benzooxazolyl, benzothiazolyl, dihydro-2H-benzo[1,4]oxazinyl, dihydro-3H-benzo[1,4]oxazinyl, dihydro-2H-benzo[1,4]thiazinyl, dihydro-1H-pyrido[2,3-b][1,4]oxazinyl, imidazo[1,2-a]pyridyl, imidazo[1,5-a]pyridyl, indazolyl, indolyl, isoquinolyl, [1,5]naphthyridyl, phenyl, phthalazinyl, pyridyl, pyrimidinyl, 1H-pyrrolo[2,3-b]pyridyl, 1 H-pyrrolo[2,3-c]pyridyl, 1H-pyrrolo[3,2-b]pyridyl, quinazolinyl, quinolyl, quinoxalinyl, tetrahydro-imidazo[1,2-a]pyridyl, tetrahydroimidazo[1,5-a]pyridyl, tetrahydroisoquinolyl, tetrahydroquinolyl, tetrahydroquinoxalinyl, [1,2,3]triazolo[1,5-a]pyridyl or [1,2,4]triazolo[4,3-a]pyridyl, each of which is substituted by 1-4 acyl-C₁₋₈-alkoxy-C₁₋₈-alkoxy, acyl-C₁₋₈-alkoxy-C₁₋₈-alkyl, (N-acyl)-C₁₋₈-alkoxy-C₁₋₈-alkylamino, C₁₋₈-alkanoyl, C₁₋₈-alkoxy, C₁₋₈-alkoxy-C₁₋₈-alkanoyl, C₁₋₈-alkoxy-C₁₋₈-alkoxy, C₁₋₈-alkoxy-C₁₋₈-alkoxy-C₁₋₈-alkyl, C₁₋₈-alkoxy-C₁₋₈-alkyl, (N-C₁₋₈-alkoxy)-C₁₋₈-alkylaminocarbonyl-C₁₋₈-alkoxy, (N-C₁₋₈-alkoxy)-C₁₋₈-alkylaminocarbonyl-C₁₋₈-alkyl, C₁₋₈-alkoxy-C₁₋₈-alkyl-carbamoyl, C₁₋₈-alkoxy-C₁₋₈-alkyl-carbonylamino, C₁₋₈-alkoxy-C₁₋₈-alkyl-heterocyclyl, C₁₋₈-alkoxy-carbonyl, C₁₋₈-alkoxycarbonyl-C₁₋₈-alkoxy, C₁₋₈-alkoxycarbonyl-C₁₋₈-alkyl, C₁₋₈-alkoxycarbonylamino, C₁₋₈-alkoxycarbonylamino-C₂₋₈-alkoxy, C₁₋₈-alkoxycarbonyl-amino-C₁₋₈-alkyl, C₁₋₈-alkyl, (N-C₁₋₈-alkyl)-C₁₋₈-alkoxy-C₁₋₈-alkyl-carbamoyl, (N-C₁₋₈-alkyl)-C₁₋₈-alkoxy-C₁₋₈-alkylcarbonylamino, (N-C₁₋₈-alkyl)-C₁₋₈-alkoxy-carbonylamino, (N-C₁₋₈-alkyl)-C₁₋₈-alkylcarbonylamino-C₂₋₈-alkoxy, (N-C₁₋₈-alkyl)-C₁₋₈-alkylcarbonylamino-C₁₋₈-alkyl, (N-C₁₋₈-alkyl)-C₁₋₈-alkylsulfonylamino-C₂₋₈-alkoxy, (N-C₁₋₈-alkyl)-C₁₋₈-alkylsulfonylamino-C₁₋₈-alkyl, C₁₋₈-alkyl-amidinyl, C₁₋₈-alkylamino-C₂₋₈-alkoxy, di-C₁₋₈-alkylamino-C₂₋₈-alkoxy, C₁₋₈-alkyl-amino-C₁₋₈-alkyl, di-C₁₋₈-alkylamino-C₁₋₈-alkyl, C₁₋₈-alkylaminocarbonyl-C₁₋₈-alkoxy, di-C₁₋₈-alkylaminocarbonyl-C₁₋₈-alkoxy, C₁₋₈-alkylaminocarbonyl-C₁₋₈-alkoxy-C₁₋₈-alkyl, C₁₋₈-alkyl-aminocarbonyl-C₁₋₈-alkyl, di-C₁₋₈-alkylaminocarbonyl-C₁₋₈-alkyl, C₁₋₈-alkylaminocarbonyl-amino-C₂₋₈-alkoxy, C₁₋₈-alkylaminocarbonylamino-C₁₋₈-alkyl, C₁₋₈-alkylcarbonylamino, C₁₋₈-alkylcarbonylamino-C₂₋₈-alkoxy, C₁₋₈-alkylcarbonylamino-C₁₋₈-alkyl, C₁₋₈-alkylcarbonyloxy-C₂₋₈-alkoxy, C₁₋₈-alkylcarbonyloxy-C₁₋₈-alkyl, C₁₋₈-alkylsulfonyl, C₁₋₈-alkylsulfonyl-C₁₋₈-alkoxy, C₁₋₈-alkylsulfonyl-C₁₋₈-alkyl, C₁₋₈-alkylsulfonylamino-C₂₋₈-alkoxy, C₁₋₈-alkylsulfonylamino-C₁₋₈-alkyl, optionally N-mono- oder N,N-di-C₁₋₈-alkylated amino, aryl-C₀₋₈-alkoxy, aryl-C₀₋₈-alkyl, optionally N-mono- oder N,N-di-C₁₋₈-alkylated carbamoyl-C₀₋₈-alkoxy, carboxy-C₁₋₈-alkoxy, carboxy-C₁₋₈-alkoxy-C₁₋₈-alkyl, carboxy-C₁₋₈-alkyl, C₁₋₈-alkyl, cyano-C₂₋₈-alkoxy, cyano-C₁₋₈-alkyl, C₃₋₈-cycloalkyl-C₁₋₈-alkoxy, C₃₋₈-cycloalkyl-carbamoyl-C₀₋₈-alkyl, C₃₋₈-cycloalkylcarbonyl-amino-C₂₋₈-alkoxy, C₃₋₈-cycloalkylcarbonylamino-C₁₋₈-alkyl, O,N-dimethylhydroxylamino-C₁₋₈-alkyl, halogen, halogen-C₁₋₈-alkoxy, halogen-C₁₋₈-alkyl, heterocydyl-C₀₋₈-alkoxy, heterocyclyl-C₀₋₈-alkyl, heterocyclyl-carbonyl, hydroxy-C₂₋₈-alkoxy-C₁₋₈-alkoxy, hydroxy-C₂₋₈-alkoxy-C₁₋₈-alkyl, hydroxy-C₁₋₈-alkyl, O-methyloximyl-C₁₋₈-alkyl, oxide or oxo.

Examples of alkyl and alkoxy radicals, which may be linear or branched, are C₁₋₈-alkyl and C₁₋₈-alkoxy radicals such as methyl, ethyl, n-propyl, isopropyl, n-butyl, isobutyl, sec-butyl, tert-butyl, pentyl, hexyl, and methoxy, ethoxy, propoxy, isopropoxy, butoxy, isobutoxy, sec-butoxy and tert-butoxy respectively, in addition C₀-alkoxy designates -O-. C₁₋₈-alkylenedioxy radicals are preferably methylenedioxy, ethylenedioxy and propylenedioxy. Examples of C₁₋₈-alkanoyl radicals, which may be linear or branched, are acetyl, propionyl and butyryl.

Cycloalkyl is a saturated, cyclic hydrocarbon radical having 3-12 carbon atoms, i.e. cyclopropyl, cyclobutyl, cyclopentyl, cyclohexyl, cycloheptyl, bicyclo[2.2.1]heptyl, cyclooctyl, bicyclo[2.2.2]octyl and adamantyl. C₁₋₈-alkylene radicals, which may be linear or branched, are, for example, methylene, ethylene, 1-methylmethylene, propylene, methylethylene, 1-ethylmethylene, 1,1-dimethylmethylene, 2-methylpropylene, 2-methylbutylene, 2-methylbutyl-2-ene, butyl-2-ene, butyl-3-ene, propyl-2-ene, tetra-, penta- and hexamethylene. C₂₋₈-alkenylene radicals, which may be linear or branched, are, for example, vinylene and propenylene. C₂₋₈-alkynylene radicals, which may be linear or branched, are, for example, ethynylene; acyl radicals are alkanoyl radicals, preferably C₁₋₈-alkanoyl radicals, or aroyl radicals such as benzoyl.

Aryl denotes mono- or polycyclic aromatic radicals which may be mono- or polysubstituted, for example phenyl, substituted phenyl, naphthyl or substituted naphthyl. Examples of substituents on such aryl radicals are acetamidinyl-C₁₋₈-alkyl, acyl-C₁₋₈-alkoxy-C₁₋₈-alkyl, (N-acyl)-C₁₋₈-alkoxy-C₁₋₈-alkylamino, C₂₋₈-alkenyl, C₂₋₈-alkenyloxy, C₁₋₈-alkoxy, C₁₋₈-alkoxy-C₁₋₈-alkoxy, C₁₋₈-alkoxy-C₁₋₈-alkoxy-C₁₋₈-alkyl, C₁₋₈-alkoxy-C₁₋₈-alkyl, (N-C₁₋₈-alkoxy)-C₁₋₈-alkyl-aminocarbonyl-C₁₋₈-alkoxy, (N-C₁₋₈-alkoxy)-C₁₋₈-alkylaminocarbonyl-C₁₋₈-alkyl, C₁₋₈-alkoxy-C₁₋₈-alkyl-carbamoyl, C₁₋₈-alkoxy-C₁₋₈-alkyl-carbonyl, C₁₋₈-alkoxy-C₁₋₈-alkyl-carbonylamino, C₁₋₈-alkoxy-C₁₋₈-alkyl-heterocydyl, 2-C₁₋₈-alkoxy-C₁₋₈-alkyl-4-oxo-lmldazol-1-yl, 6-alkoxy-aminocarbonyl-C₁₋₈-alkoxy, C₁₋₈-alkoxy-aminocarbonyl-C₁₋₈alkyl, C₁₋₈-alkoxycarbonyl, C₁₋₈-alkoxycarbonyl-C₁₋₈-alkoxy, C₁₋₈-alkoxycarbonyl-C₁₋₈-alkyl, C₁₋₈-alkoxycarbonylamino-C₁₋₈-alkoxy, C₁₋₈-alkoxycarbonylamino-C₁₋₈-alkyl, C₁₋₈-alkoxycarbonylphenyl, C₁₋₈-alkyl, (N-C₁₋₈-alkyl)-C₁₋₈-alkoxy-C₁₋₈-alkyl-carbamoyl, (N-C₁₋₈-alkyl)-C₁₋₈-alkoxy-C₁₋₈-alkyl-carbonylamino, (N-C₁₋₈-alkyl)-C₁₋₈-alkoxy-carbonylamino, (N-C₁₋₈-alkyl)-C₀₋₈-alkylcarbonylamino-C₁₋₈-alkoxy, (N-C₁₋₈-alkyl)-C₀₋₈-alkylcarbonylamino-C₁₋₈-alkyl, (N-C₁₋₈-alkyl)-C₁₋₈-alkylsulfonylamino-C₁₋₈-alkoxy, (N-C₁₋₈-alkyl)-C₁₋₈-alkylsulfonylamino-C₁₋₈-alkyl, C₁₋₈-alkyl-amidinyl, optionally N-mono or N,N-di-C₁₋₈-alkylated amino, C₁₋₈-alkylamino-C₂₋₈-alkoxy, di-C₁₋₈-alkylamino-C₂₋₈-alkoxy, C₁₋₈-alkylamino-C₁₋₈-alkyl, di-C₁₋₈-alkylamino-C₁₋₈-alkyl, C₁₋₈-alkylaminocarbonyl-C₁₋₈-alkoxy, di-C₁₋₈-alkylaminocarbonyl-C₁₋₈-alkoxy, C₁₋₈-alkylaminocarbonyl-C₁₋₈-alkoxy-C₁₋₈-alkyl, C₁₋₈-alkylaminocarbonyl-C₁₋₈-alkyl, di-C₁₋₈-alkylaminocarbonyl-C₁₋₈-alkyl, C₁₋₈-alkylaminocarbonyl-amino-C₁₋₈-alkoxy, C₁₋₈-alkylaminocarbonylamino-C₁₋₈-alkyl, C₁₋₈-alkyl-carbamoyl, di-C₁₋₈-alkyl-carbamoyl, C₀₋₈-alkylcarbonylamino, C₀₋₈-alkylcarbonylamino-C₁₋₈-alkoxy, C₀₋₈-alkyl-carbonylamino-C₁₋₈-alkyl, C₁₋₈-alkylcarbonyloxy, C₁₋₈-alkylcarbonyloxy-C₁₋₈-alkoxy, C₁₋₈-alkylcarbonyloxy-C₁₋₈-alkyl, C₁₋₈-alkylenedioxy, C₁₋₈-alkyl-sulfonyl, C₁₋₈-alkylsulfonyl-C₁₋₈-alkoxy, C₁₋₈-alkylsulfonyl-C₁₋₈-alkyl, C₁₋₈-alkylsulfonylamino-C₁₋₈-alkoxy, C₁₋₈-alkylsulfonyl-amino-C₁₋₈-alkyl, aryl-C₁₋₈-alkanoyl, aryl-C₀₋₈-alkoxy, aryl-C₀₋₈-alkyl, arylamino, aryloxy, arylthio, benzoyloxy-C₁₋₈-alkoxy, benzyloxy, carbamoyl-C₀₋₈-alkoxy, carbamoyl-C₀₋₈-alkyl, carboxy, carboxy-C₁₋₈-alkoxy, carboxy-C₁₋₈-alkoxy-C₁₋₈-alkyl, carboxy-C₁₋₈-alkyl, cyano, cyano-C₁₋₈-alkoxy, cyano-C₁₋₈-alkyl, C₃₋₈-cycloalkyl-C₁₋₈-alkanoyl, C₃₋₈-cycloalkylcarbonyl-amino-C₁₋₈-alkoxy, C₃₋₈-cycloalkylcarbonylamino-C₁₋₈-alkyl, cyclopropyl-C₁₋₈-alkoxy, cyclopropyl-C₁₋₈-alkyl, O,N-dimethylhydroxylamino-C₁₋₈-alkyl, dioxolanyl-C₁₋₈-alkoxy, halogen, halogen-C₁₋₈-alkoxy, halogen-C₁₋₈-alkyl, heterocyclyl, heterocyclyl-C₁₋₈-alkanoyl, heterocyclyl-C₁₋₈-alkoxy, heterocyclyl-C₁₋₈-alkoxy-C₁₋₈-alkoxy, heterocyclyl-C₁₋₈-alkoxy-C₁₋₈-alkyl, heterocyclyl-C₁₋₈-alkyl, heterocyclylamino, heterocyclyloxy, heterocyclylthio, hydroxy, hydroxy-C₂₋₈-alkoxy, hydroxy-C₂₋₈-alkoxy-C₁₋₈-alkoxy, hydroxy-C₂₋₈-alkoxy-C₁₋₈-alkyl, hydroxy-C₁₋₈-alkyl, (N-hydroxy)-C₁₋₈-alkylaminocarbonyl-C₁₋₈-alkoxy, (N-hydroxy)-C₁₋₈-alkylaminocarbonyl-C₁₋₈-alkyl, hydroxy-C₁₋₈-alkylphenyl, (N-hydroxy)-aminocarbonyl-C₁₋₈-alkoxy, (N-hydroxy)-amino-carbonyl-C₁₋₈-alkyl, hydroxybenzyloxy, methylendioxybenzyloxy, methoxybenyloxy, O-methyl-oximyl-C₁₋₈-alkyl, nitro, 2-oxo-oxazolidinyl-C₁₋₈-alkoxy, 2-oxo-oxazolidinyl-C₁₋₈-alkyl or pyridyl-carbonylamino-C₁₋₈-alkyl.

The term heterocyclyl denotes mono- or polycyclic, saturated and unsaturated heterocyclic radicals having from 1 to 4 nitrogen and/or 1 or 2 sulfur or oxygen atoms and which may be mono- or polysubstituted, especially mono- ,di- or trisubstituted. Additionally, the term heterocyclyl includes the above mentioned oxo-substituted radicals. Examples of unsaturated heterocyclyl radicals are benzo[1,3]dioxolyl, benzofuranyl, benzoimidazolyl, benzooxazolyl, benzothiazolyl, benzo[b]thienyl, quinazolinyl, quinolyl, quinoxalinyl, dihydrobenzofuranyl, 1,3-dihydro-benzoimidazol, 3,4-dihydro-2H-benzo[1,4]oxazinyl, dihydro-3H-benzo[1,4]oxazinyl, 1,4-dihydro-benzo[d][1,3]oxazin, dihydro-2H-benzo[1,4]thiazinyl, 3,4-dihydro-1H-quinazolin, 3,4-dihydro-1H-quinolin, 2,3-dihydroindolyl, dihydro-1H-pyrido[2,3-b][1,4]oxazinyl, 1,1-dioxo-dihydro-2H-benzo[1,4]thiazinyl, furyl, imidazolyl, imidazo[1,5-a]pyridinyl, imidazo[1,2-a]pyrimidinyl, indazolyl, indolyl, isobenzofuranyl, isoquinolyl, [1,5]naphthyridyl, oxazolyl, 1-oxido-pyridyl, 2-oxo-benzoimidazolyl, 3-oxo-4H-benzo[1,4]oxazinyl, 2-oxo-benzooxazolyl, 3-oxo-4H-benzo[1,4]thiazinyl, 2-oxo-dihydro-benzo[e][1,4]diazepinyl, 2-oxo-1,3-dihydro-benzoimidazol, 2-oxo-dihydro-benzo-[d][1,3]oxazinyl, 2-oxo-3,4-dihydro-1H-quinazolin, 2-oxo-3,4-dihydro-1H-quinolin, 4-oxo-dihydro-imidazolyl, 2-oxo-1,3-dihydroindolyl, 1-oxo-3H-isobenzofuranyl, 2-oxo-1H-pyrido[2,3-b][1,4]oxazinyl, 2-oxo-1,3,4,5-tetrahydro-benzo[b]azepin, 2-oxo-tetrahydro-benzo[e][1,4]diazepinyl, 4-oxo-3H-thieno[2,3-d]pyrimidinyl, 5-oxo-4H-[1,2,4]triazinyl, phthalazinyl, pyranyl, pyrazinyl, pyrazolyl, pyridyl, pyrimidinyl, 1H-pyrrolizinyl, pyrrolo[3,2-c]pyridinyl, pyrrolo[2,3-c]pyridinyl, pyrrolo[3,2-b]pyridinyl, 1H-pyrrolo[2,3-b]pyridyl, pyrrolyl, 1,3,4,5-tetrahydro-benzo[b]azepin, tetrahydroquinolinyl, tetrahydroquinoxalinyl, tetrahydroisoquinolinyl, thiazolyl, thienyl, triazinyl, triazolyl, 1,1,3-trioxo-dihydro-2H-1lambda*6*-benzo[1,4]thiazinyl, [1,2,3]triazolo[1,5-a]pyridinyl or [1,2,4]triazolo[4,3-a]pyridinyl.

The term saturated heterocyclyl denotes 3-16 membered mono- or bicyclic, saturated heterocyclic radicals having from 1 to 4 nitrogen and/or 1 or 2 sulfur or oxygen atoms. Prefered are 3-8 membered, especially preferred 5- or 6-membered monocyclic radicals, which may be condensed to a 3-8 membered, carbocyclic or heterocyclic ring. Another preferred group of saturated heterocyclic radicals are bicyclic radicals possessing a spirocyclic or bridged ring skeleton. Preferred heterocyclic radicals are possessing per ring 1 nitrogen, oxygen or sulfur atom, 1-2 nitrogen atoms and 1-2 oxygen atoms or 1-2 nitrogen atoms and 1-2 sulfur atoms, whereby, per ring, at least 1 carbon atom, preferentially 1-7 carbon atoms are present.

Examples for saturated heterocyclyl radicals are azepanyl, azetidinyl, aziridinyl, 3,4-dihydroxy-pyrrolidinyl, 2,6-dimethylmorpholinyl, 3,5-dimethylmorpholinyl, dioxanyl, [1,4]dioxepanyl, dioxolanyl, 4,4-dioxothiomorpholinyl, dithianyl, dithiolanyl, 2-hydroxymethylpyrrolidinyl, 4-hydroxypiperidinyl, 3-hydroxypyrrolidinyl, 4-methylpiperazinyl, 1-methylpiperidinyl, 1-methyl-pyrrolidinyl, morpholinyl, oxathianyl, oxepanyl, 2-oxo-azepanyl, 2-oxo-imidazolidinyl, 2-oxo-oxazolidinyl, 2-oxo-piperidinyl, 4-oxo-piperidinyl, 2-oxo-pyrrolidinyl, 2-oxo-tetrahydro-pyrimidinyl, 4-oxo-thiomorpholinyl, piperazinyl, piperidinyl, pyrrolidinyl, tetrahydrofuranyl, tetrahydropyranyl, tetrahydrothiophenyl, tetrahydrothiopyranyl, thiepanyl or thiomorpholinyl.

Examples for saturated bicyclic heterocyclyl radicals are 2,5-dioxa-bicyclo[4.1.0]heptanyl, 2-oxa-bicyclo[2.2.1]heptanyl, 2-oxa-bicyclo[4.1.0]heptanyl, 3-oxa-bicyclo[4.1.0]heptanyl, 7-oxa-bicyclo[2.2.1]heptanyl, 2-oxa-bicyclo[3.1.0]hexanyl, 3-oxa-bicyclo[3.1.0]hexanyl, 1-oxa-spiro[2.5]octanyl, 6-oxa-spiro[2.5]octanyl or 3-oxa-bicyclo[3.3.1]nonanyl.

Heterocyclyl radicals may be unsubstituted or mono- or polysubstituted, for example mono-or disubstituted. Examples of substituents on such heterocyclyl radicals are C₁₋₈-alkanoyl, C₂₋₈ -alkenyl, C₂₋₈-alkynyl, C₁₋₈-alkoxy, C₁₋₈-alkoxy-C₁₋₈-alkoxy, C₁₋₈-alkoxy-C₁₋₈-alkyl, C₁₋₈-alkoxy-carbonylamino-C₂₋₈-alkoxy, C₁₋₈-alkoxycarbonylamino-C₀₋₈-alkyl, C₁₋₈-alkyl, C₀₋₈-alkylcarbonyl-amino-C₂₋₈-alkoxy, C₀₋₈-alkylcarbonylamino-C₀₋₈-alkyl, C₁₋₈-alkylcarbonyloxy, C₁₋₈-alkylene-dioxy, optionally N-mono or N,N-di-C₁₋₈-alkylated amino, aryl, optionally N-mono or N,N-di-C₁₋₈-alkylated carbamoyl, optionally esterified carboxy, cyano, C₃₋₈-cycloalkoxy, halogen, heteroaryl, heterocyclyl, hydroxy, nitro, oxid, oxo, polyhalogen-C₁₋₈-alkoxy or polyhalogen-C₁₋₈-alkyl.

The term polyhydroxyalkyl denotes C₁₋₇-alkyl radicals which may be substituted by 2-6 hydroxyl groups, for example glyceryl, arabityl, sorbityl, etc.

Halogen or halo denotes, for example, fluorine, chlorine or bromine, or a radical singly, multiply or fully substituted by fluorine, chlorine or bromine.

In case of a polysubstituted radical, each individual substituent is selected independently of the other(s).

The radical (A) is a mono- or di--peptidic residue derived from the 20 natural amino acids, formally by elimination of the OH-group of the C-terminal end, which each, except for the residue derived from glycine only, independently of the others, may be present either in the (D)-, (L)- or racemic (D,L)- configuration, but preferably in the L-form. Examples of such amino acids (mono-peptides) are valine, glycine, alanine, leucine, isoleucine, phenylalanine, tyrosine, aspartic acid and glutamic acid. Examples of groups of the formula (A), the asterix indicating the bond to the rest of the molecule of formula (I), are thus:

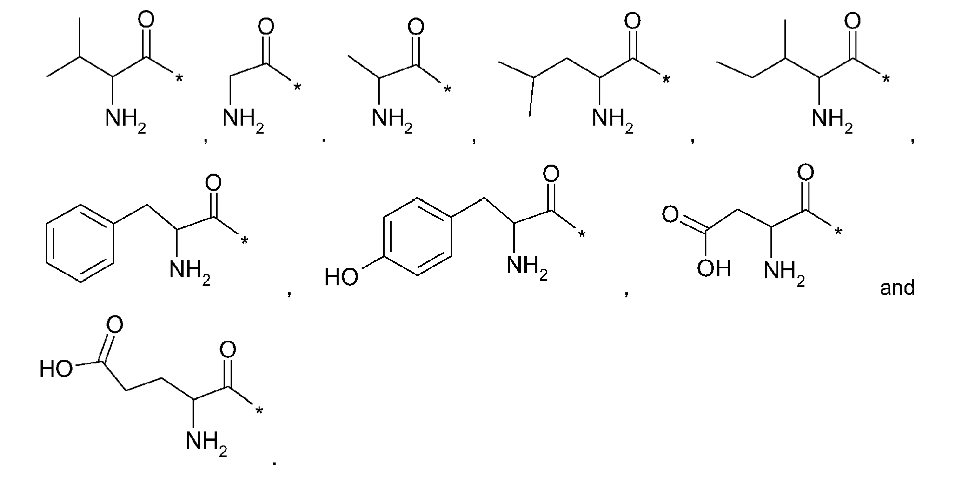

Examples of such di-peptides are glycylvaline, glycylleucine and valylvaline. Examples of radicals (A), the asterix indicating the bond to the rest of the molecule of formula (I), are thus:

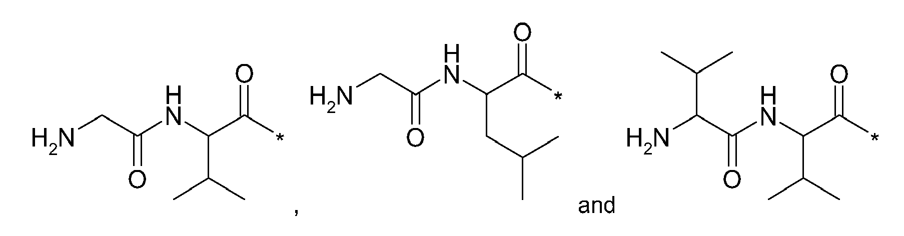

Salts are primarily the pharmaceutically usable or nontoxic salts of compounds of formula (I). The term "pharmaceutically useable salts" encompasses salts with inorganic or organic acids, such as hydrochloric acid, hydrobromic acid, nitric acid, sulfuric acid, phosphoric acid, citric acid, formic acid, maleic acid, acetic acid, succinic acid, tartaric acid, methanesulfonic acid, p-toluenesulfonic acid and the like.

Salts of compounds having salt-forming groups are in particular acid addition salts, salts with bases, or, in the presence of a plurality of salt-forming groups, in some cases also mixed salts or internal salts.

Such salts are formed, for example, from compounds of formula (I) with an acidic group, for example a carboxyl or sulfonyl group, and are, for example, the salts thereof with suitable bases such as non-toxic metal salts derived from metals of group Ia, Ib, IIa and IIb of the Periodic Table of the Elements, for example alkali metal, in particular lithium, sodium, or potassium, salts, alkaline earth metal salts, for example magnesium or calcium salts, and also zinc salts and ammonium salts, including those salts which are formed with organic amines, such as optionally hydroxy-substituted mono-, di- or trialkylamines, in particular mono-, di- or tri(lower alkyl)amines, or with quaternary ammonium bases, e.g. methyl-, ethyl-, diethyl- or triethylamine, mono-, bis- or tris(2-hydroxy(lower alkyl))amines, such as ethanol-, diethanol- or triethanolamine, tris(hydroxymethyl)methylamine or 2-hydroxy-tert-butylamine, N,N-di(lower alkyl)-N-(hydroxy(lower alkyl))amine, such as N,N-di-N-dimethyl-N-(2-hydroxyethyl)amine, or N-methyl-D-glucamine, or quaternary ammonium hydroxides such as tetrabutylammonium hydroxide. The compounds of formula (I) having a basic group, for example an amino group, may form acid addition salts, for example with suitable inorganic acids, e.g. hydrohalic acid such as hydrochloric acid, hydrobromic acid, sulfuric acid with replacement of one or both protons, phosphoric acid with replacement of one or more protons, e.g. orthophosphoric acid or metaphosphoric acid, or pyrophosphoric acid with replacement of one or more protons, or with organic carboxylic, sulfonic or phosphonic acids or N-substituted sulfamic acids, e.g. acetic acid, propionic acid, glycolic acid, succinic acid, maleic acid, hydroxymaleic acid, methylmaleic acid, fumaric acid, malic acid, tartaric acid, gluconic acid, glucaric acid, glucuronic acid, citric acid, benzoic acid, cinnamic acid, mandelic acid, salicylic acid, 4-amino-salicylic acid, 2-phenoxybenzoic acid, 2-acetoxybenzoic acid, embonic acid, nicotinic acid, isonicotinic acid, and also amino acids, for example the alpha-amino acids mentioned above, and also methanesulfonic acid, ethanesulfonic acid, 2-hydroxyethanesulfonic acid, ethane-1,2-disulfonic acid, benzenesulfonic acid, 4-methylbenzenesulfonic acid, naphthalene-2-sulfonic acid, 2- or 3-phosphoglycerate, glucose 6-phosphate, N-cyclohexylsulfamic acid (with formation of the cyclamates) or with other acidic organic compounds such as ascorbic acid. Compounds of formula (I) having acidic and basic groups may also form internal salts.

Salts obtained may be converted to other salts in a manner known per se, acid addition salts, for example, by treating with a suitable metal salt such as a sodium, barium or silver salt, of another acid in a suitable solvent in which an inorganic salt which forms is insoluble and thus separates out of the reaction equilibrium, and base salts by release of the free acid and salt reformation.

The compounds of formula (I), including their salts, may also be obtained in the form of hydrates or include the solvent used for the crystallization.

For the isolation and purification, pharmaceutically unsuitable salts may also find use.

The compounds of formula (I) also include those compounds in which one or more atoms are replaced by their stable, non-radioactive isotopes; for example a hydrogen atom by deuterium.

Preferred procedures for the preparation of substituted amino alcohols have been described in EP 678503, WO 2005/070870, WO 2005/070871, WO 2005/070877 and WO 2005/090305, The compounds of formula (I) and salts thereof may be prepared in a similar manner to the preparation processes disclosed in the literature. Details on the specific preparation variants can be taken from the examples.

The compounds of formula (I) have at least two asymmetric carbon atoms and may therefore be in the form of optically pure diastereomers, diastereomeric mixtures, diastereomeric racemates, mixtures of diastereomeric racemates or as meso compounds. The invention encompasses all of these forms. Diastereomeric mixtures, diastereomeric racemates or mixtures of diastereomeric racemates may be separated by customary procedures, for example by column chromatography, thin-layer chromatography, HPLC and the like.

The compounds of formula (I) may also be prepared in optically pure form. The separation into antipodes can be effected by procedures known per se, either preferably at an earlier synthetic stage by salt formation with an optically active acid, for example (+)- or (-)-mandelic acid and separation of the diastereomeric salts by fractional crystallization, or preferably at a relatively late stage by derivatizing with a chiral auxiliary building block, for example (+)- or (-)-camphanoyl chloride, and separation of the diastereomeric products by chromatography and/or crystallization and subsequent cleavage of the bonds to give the chiral auxiliary. The pure diastereomeric salts and derivatives may be analysed to determine the absolute configuration of the piperidine present with common spectroscopic procedures, and X-ray spectroscopy on single crystals constitutes a particularly suitable procedure.

It is possible for the configuration at individual chiral centres in a compound of formula (I) to be inverted selectively. For example, the configuration of asymmetric carbon atoms which bear nucleophilic substituents, such as amino or hydroxyl, may be inverted by second-order nucleophilic substitution, if appropriate after conversion of the bonded nucleophilic substituent to a suitable nucleofugic leaving group and reaction with a reagent which introduces the original substituents, or the configuration at carbon atoms having hydroxyl groups can be inverted by oxidation and reduction, analogously to the process in the European patent application EP-A-0 236 734. Also advantageous is the reactive functional modification of the hydroxyl group and subsequent replacement thereof by hydroxyl with inversion of configuration.

The compound groups mentioned below are not to be regarded as closed, but rather parts of these compound groups may be exchanged with one another or with the definitions given above or omitted in a sensible manner, for example to replace general by more specific definitions. The definitions are valid in accordance with general chemical principles, such as, for example, the common valences for atoms.

Preferred compounds according to the invention are those of the general formula (lA) 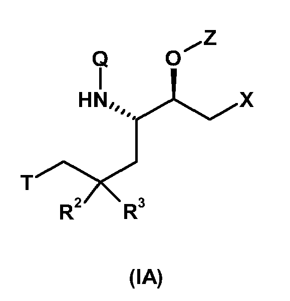 and the salts thereof, preferably the pharmaceutically acceptable salts thereof;
in which R², R³, Q, T, X and Z have the meaning stated above for the compounds of the formula (I).

A further preferred group of compounds of the formula (I) or more preferably of the formula (IA) are compounds and the salts thereof, preferably the pharmaceutically acceptable salts thereof; where

R¹ is aryl or a nitrogen-containing heterocyclyl, especially benzoimidazolyl, benzooxazolyl, benzothiazolyl, dihydro-2H-benzo[1,4]oxazinyl, dihydro-3H-benzo[1,4]oxazinyl, dihydro-2H-benzo[1,4]thiazinyl, dihydro-1H-pyrido[2,3-b][1,4]oxazinyl, imidazo[1,2-a]pyridyl, imidazo[1,5-a]pyridyl, indazolyl, indolyl, isoquinolyl, [1,5]naphthyridyl, phenyl, phthalazinyl, pyridyl, pyrimidinyl, 1H-pyrrolo[2,3-b]pyridyl, 1H-pyrrolo[2,3-c]pyridyl, 1H-pyrrolo[3,2-b]pyridyl, quinazolinyl, quinolyl, quinoxalinyl, tetrahydroimidazo[1,2-a]pyridyl, tetrahydroimidazo[1,5-a]pyridyl, tetrahydroisoquinolyl, tetrahydroquinolyl, tetrahydroquinoxalinyl, [1,2,3]triazolo[1,5-a]pyridyl or [1,2,4]triazolo[4,3-a]pyridyl, which is substituted by 1-4 C₁₋₈-alkoxy, C₁₋₈-alkoxy-C₁₋₈-alkoxy, C₁₋₈-alkoxy-C₁₋₈-alkyl, C₁₋₈-alkoxycarbonylamino or C₁₋₈-alkyl.

A group of especially preferred compounds of the formula (I) or more preferably of the formula (IA) are compounds and the salts thereof, preferably the pharmaceutically acceptable salts thereof; where

R⁴ is optionally substituted aryl-C₀₋₈-alkyl, optionally substituted C₃₋₈-cycloalkyl-C₀₋₈-alkyl or optionally substituted heterocyclyl-C₀₋C₆-alkyl,
whereby,
each of the residues, mentioned above for R⁴, bearing a hydroxy-group is optionally substituted at that hydroxy-group by a radical (A).

A group of especially preferred compounds of the formula (I) or more preferably of the formula (IA) are compounds and the salts thereof, preferably the pharmaceutically acceptable salts thereof; where
A) X is -NR⁵-C(O)R⁴, -Alk-C(O)-NR⁵R⁴ or -NR⁸R⁹, where Alk represents C₁₋₈-alkylene; and
   T is R¹, R¹-C(O)- or R¹-C(O)-NR⁵-; and
   R¹ is a substituted benzoimidazolyl, 3,4-dihydro-2H-benzo[1,4]oxazinyl, indazolyl, indolyl or pyridyl; or
B) X is -NR⁵-C(O)R⁴ or -NR⁸R⁹, where Alk represents C₁₋₈-alkylene; and
   T is R¹, R¹-C(O)- or R¹-C(O)-NR⁵-; and
   R¹ is substituted phenyl; or
C) X is -Alk-C(O)-NR⁵R⁴, where Alk represents C₁₋₈-alkylene and R⁴ is tetrahydropyranyl-C₀-C₄-alkyl; and
   T is R¹; and
   R¹ is substituted phenyl;
R² und R³ are, independently from each other, hydrogen or C₁₋₆-alkyl or, together with the carbon atom they are linked to, are C₃₋₈-cycloalkyl;
R⁴ is optionally substituted aryl-C₀₋₈-alkyl, optionally substituted C₃₋₈-cycloalkyl-C₀₋₈-alkyl or optionally substituted heterocyclyl-C₀₋C₆-alkyl,
whereby,
each of the residues, mentioned above for R⁴, bearing a hydroxy-group is optionally substituted at that hydroxy-group by a radical (A);
R⁵ is hydrogen or C₁₋₈-alkyl;
R⁶ is optionally carboxy- or hydroxy-substituted C₁₋₈-alkyl or aryl-C₀₋₈-alkyl;
R⁷ is C₁₋₈-alkyl;
R⁸ and R⁹, together with the nitrogen atom to which they are bonded, are a 5-7-membered ring;
(A) represents a mono- or di-peptidic residue of one or two of the 20 natural amino acids, said residue being formally obtained from the mono- or di-peptide by elimination of the
OH-group from the C-terminal end;
Q is hydrogen or represents a radical (A) or a group of the formula -C(O)OCHR⁶OC(O)R⁸;
Z is hydrogen or represents a radical (A);
and whereby
(i) a radical (A) is present in at least one of R⁴, Q or Z; or else
(ii) at least Q is a group of the formula -C(O)OCHR⁶OC(O)R⁷.

The compounds of formula (I), or preferably of formula (IA), and their pharmaceutically useable salts have inhibiting action on the natural enzyme renin. The latter passes from the kidneys into the blood and there brings about the cleavage of angiotensinogen to form the decapeptide angiotensin I which is then cleaved in the lung, the kidneys and other organs to the octapeptide angiotensin II. Angiotensin II increases the blood pressure both directly by arterial constriction and indirectly by the release of the hormone aldosterone which inhibits the release of the sodium ion from the adrenal glands, which is associated with a rise in the extracellular liquid volume. This rise can be attributed to the action of angiotensin II itself or of the heptapeptide angiotensin III formed therefrom as a cleavage product. Inhibitors of the enzymatic activity of renin bring about a reduction in the formation of angiotensin I and, as a consequence thereof, the formation of a smaller amount of angiotensin II. The reduced concentration of this active peptide hormone is the immediate cause of the hypotensive action of renin inhibitors.

One experimental procedure of detecting the action of transporters, in particular PEPT1, is by means of *in vitro* tests, in which inhibition of [¹⁴C]Gly-Sar uptake is measured. One *in vitro* test which is used is the one according to Faria et al. J. Med. Chem. 49(12), 3636-3644, (2006).
This assay uses a stably transfected cell line which expresses the rPepT1 transporter. [¹⁴C]Gly-Sar (10 µM) is added together with an excess of an unlabelled competitor (0.1 - 9 mM) onto the monolayer cell culture grown in microtiter plates. The uptake of [¹⁴C]Gly-Sar is determined in a subsequent radioimmunoassay measuring the [14C]Gly-Sar content in the solubilized cells. The IC₅₀ is defined as the concentration of the particular competitor which reduces the uptake of [¹⁴C]Gly-Sar by 50%. The IC₅₀ values are estimated by nonlinear regression analysis. Michaelis-Menten-like kinetic parameters (Kₘ, Vₘₐₓ) are determined by nonlinear curve fitting of specific uptake data to the following equation: V₀ = Vₘₐₓ[S]/(Kₘ+[S]), where V₀ is the initial uptake velocity, Vₘₐₓ is the maximal uptake velocity at saturating substrate concentrations, Kₘ is a constant analogous to the Michaelis-Menten constant and S is the substrate concentration. The compounds of the present invention which are substrates of PEPT1 show inhibitory effects in the in vitro system at minimal concentrations of about 10⁻² to about 10⁻⁵ mol/l.

The pharmacokinetic properties of the compounds described herein can be tested *in vivo* using the following protocol: The investigations take place in pre-catheterized (carotid artery) male rats (300 g ±20%) that can move freely throughout the study. The compound is administered intravenously and orally (gavage) in separate sets of animals. The applied doses for oral administration may range from 0.5 to 50 mg/kg body weight; the doses for intravenous administration may range from 0.5 to 20 mg/kg body weight. Blood samples are collected through the catheter before compound administration and over the subsequent 24-hour period using an automated sampling device (AccuSampler, DiLab Europe, Lund, Sweden). Plasma levels of the compound are determined using a validated LC-MS analytical method. The pharmacokinetic analysis is performed on the plasma concentration-time curves after averaging all plasma concentrations across time points for each route of administration. Typical pharmacokinetics parameters to be calculated include: maximum concentration (Cₘₐₓ), time to maximum concentration (tₘₐₓ), area under the curve from 0 hours to the time point of the last quantifiable concentration (AUC₀₋ₜ), area under the curve from time 0 to infinity (AUC_{0-inf}), elimination rate constant (K), terminal half-life (t_{½}), absolute oral bioavailability or fraction absorbed (F), clearance (CL), and volume of distribution during the terminal phase (Vd). The compounds of the present invention effectively increase the concentration of parent compound in plasma in the *in vivo* test described at doses of about 0.3 to about 30 mg/kg p.o.

In salt-depleted animals, renin inhibitors bring about a blood pressure decrease. Human renin differs from renin of other species. To test inhibitors of human renin, primates (marmosets, Callithrixjacchus) are used, because human renin and primate renin are substantially homologous in the enzymatically active region. One in vivo test which is used is as follows: the test compounds are tested on normotensive marmosets of both genders and having a body weight of about 350 g which are conscious, able to move freely and in their normal cages. Blood pressure and heart rate are measured using a catheter in the descending aorta and recorded radiometrically. The endogenous release of renin is stimulated by the combination of a 1-week low-salt diet with a single intra-muscular injection of furosemide (5-(aminosulfonyl)-4-chloro-2-[(2-furanylmethyl)amino]benzoic acid) (5 mg/kg). 16 hours after the injection of furosemide, the test substances are administered either directly into the femoral artery by means of an injection cannular or into the stomach by gavage as a suspension or solution, and their effect on blood pressure and heart rate was evaluated. The compounds of the present invention effectively reduce blood pressure in the *in vivo* test described at doses of about 0.003 to about 0.3 mg/kg i.v. and at doses of about 0.3 to about 30 mg/kg p.o.

The compounds of formula (I), or preferably of formula (IA), and their pharmaceutically useable salts may find use as medicaments, for example in the form of pharmaceutical preparations. The pharmaceutical preparations may be administered enterally, such as orally, for example in the form of tablets, coated tablets, sugar-coated tablets, hard and soft gelatine capsules, solutions, emulsions or suspensions, nasally, for example in the form of nasal sprays, rectally, for example in the form of suppositories, or transdermally, for example in the form of ointments or patches. The administration may also be parenteral, such as intramuscular or intravenous, for example in the form of injection solutions.

To prepare tablets, coated tablets, sugar-coated tablets and hard gelatine capsules, the compounds of formula (I), or preferably of formula (lA), and pharmaceutically useable salts thereof, may be processed with pharmaceutically inert, inorganic or organic excipients. Such excipients used, for example for tablets, coated tablets and hard gelatine capsules, may be lactose, corn starch, or derivatives thereof, talc, stearic acid or salts thereof etc.
Suitable excipients for soft gelatine capsules are, for example, vegetable oils, waxes, fats, semisolid and liquid polyols, etc.
Suitable excipients for preparing solutions and syrups are, for example, water, polyols, sucrose, invert sugar, glucose, etc.
Suitable excipients for injection solutions are, for example, water, alcohols, polyols, glycerol, vegetable oils, bile acids, lecithin, etc.
Suitable excipients for suppositories are, for example, natural or hardened oils, waxes, fats, semisolid or liquid polyols, etc.

The pharmaceutical preparations may additionally also comprise preservatives, solubilizers, viscosity-increasing substances, stabilizers, wetting agents, emulsifiers, sweeteners, colorants, flavourings, salts for altering the osmotic pressure, buffers, coatings or antioxidants. They may also comprise other therapeutically valuable substances.

The present invention further provides the use of the compounds of formula (I), or preferably of formula (IA), and the pharmaceutically useable salts thereof, in the treatment or prevention of hypertension and heart failure, and also glaucoma, cardiac infarction, kidney failure, restenoses and stroke of mammals, especially of human beings.

The compounds of formula (I), or preferably of formula (IA), and the pharmaceutically useable salts thereof, may also be administered in combination with one or more agents having cardiovascular action, for example α- and β-blockers such as phentolamine, phenoxy-benzamine, prazosin, terazosin, tolazine, atenolol, metoprolol, nadolol, propranolol, timolol, carteolol etc.; vasodilators such as hydralazine, minoxidil, diazoxide, nitroprusside, flose-quinan etc.; calcium antagonists such as amrinone, bencyclan, diltiazem, fendiline, flunarizine, nicardipine, nimodipine, perhexilene, verapamil, gallopamil, nifedipine etc.; ACE inhibitors such as cilazapril, captopril, enalapril, lisinopril etc.; potassium activators such as pinacidil; anti-serotoninergics such as ketanserin; thromboxane-synthetase inhibitors; neutral endopeptidase inhibitors (NEP inhibitors); angiotensin II antagonists; and also diuretics such as hydrochlorothiazide, chlorothiazide, acetazolamide, amiloride, bumetanide, benzthiazide, ethacrynic acid, furosemide, indacrinone, metolazone, spironolactone, triamteren, chlorthalidone etc.; sympatholytics such as methyldopa, clonidine, guanabenz, reserpine; and other agents which are suitable for the treatment of hypertension, heart failure or vascular diseases in humans and animals which are associated with diabetes or renal disorders such as acute or chronic renal failure. Such combinations may be employed separately or in preparations which comprise a plurality of components.

Further substances which can be used in combination with the compounds of formula (I), or preferably of formula (IA), are the compounds of classes (i) to (ix) on page 1 of WO 02/40007 (and also the preferences and examples further listed therein) and the substances specified on pages 20 and 21 of WO 03/027091.

The dose may vary within wide limits and has of course to be adapted to the individual circumstances in each individual case. In general, for oral administration, a daily dose of about 3 mg to about 3 g, preferably about 10 mg to about 1 g, for example about 300 mg, per adult (70 kg), divided into preferably 1-3 individual doses which may, for example, be of equal size, may be appropriate, although the upper limit specified may also be exceeded if this should be found to be appropriate; typically, children receive a lower dose according to their age and body weight.

### Examples

The examples which follow illustrate the present invention. All temperatures are reported in degrees Celsius, pressures in mbar. Unless stated otherwise, the reactions take place at room temperature. The abbreviation "Rf = xx (A)" means, for example, that the Rf value xx is obtained in the solvent system A. The ratio of the solvents relative to one another is always reported in parts by volume. Chemical names of end products and intermediates were obtained with the aid of the program AutoNom 2000 (Automatic Nomenclature).

HPLC gradient on Hypersil BDS C-18 (5 µm); column: 4 x 125 mm
- I: 90% water*/10% acetonitrile* to 0% water*/100% acetonitrile* in 5 minutes + 2.5 minutes (1.5 ml/min); *: containing 0.1 % trifluoroacetic acid
- II: 95% water*/5% acetonitrile* to 0% water*/100% acetonitrile* in 40 minutes + 2.5 minutes (0.8 ml/min); *: containing 0.1 % trifluoroacetic acid

The following abbreviations are used:
- Rf: ratio of distance which a substance travels to distance of the eluent front from the start point in thin layer chromatography
- Rt: retention time of a substance in HPLC (in minutes)
- m.p.: melting point (temperature)

### General procedure A (N-Tos-deprotection)

To a stirred solution of 0.09 mmol "tosylamide" in 10 ml of methanol are added 0.44 mmol sodiumdihydrogenphosphate and 0.90 mmol of sodium amalgam (10% Na) at room temperature. The reaction mixture is stirred for 2-18 hours, diluted with water and extracted with ethyl acetate. The organic phases are combined, washed with brine and dried over sodium sulfate. The solvent is concentrated under reduced pressure and the residue is purified by flash chromatography (SiO₂ 60F) to afford the title compound.

### General procedure B: (N-p-methoxyphenyl deprotection)

To a stirred solution of 0.5 mmol "p-methoxyaniline" in 10 ml of acetonitrile/water (1:1) is added a solution of 1.43 mol of ceric ammonium nitrate in 5.0 ml of water at 0°C. The mixture is stirred for 30 min, followed by addition of 1.0 g of sodium sulfite. After additional 30 min, the mixture is diluted with water and extracted with tert-butyl methylether. The organic phase is dried and concentrated under reduced pressure. The residue is purified by flash chromatography (SiO₂ 60F) to afford the title compound.

### General procedure C: (BH₃-reduction)

To a stirred solution of 1 mmol of "lactam" in 3 ml of tetrahydrofuran is admixed with 2 - 4 mmol of borane tetrahydrofuran (1M in tetrahydrofuran) and heated to 50°C for 2 - 8 hours.

The reaction mixture is quenched by addition of 10 ml of methanol and concentrated under reduced pressure. The title compound is obtained from the residue by means of flash chromatography (SiO₂ 60F).

### General procedure D: (amide-formation)

To a stirred solution of 1.0 mmol of "acid" and "1.0 mmol of "amin" in 20 ml of dichloromethane are added 5.0 mmol of triethylamin and 1.0 mmol of tri-propylphosphonic acid cyclic anhydride [68957-94-8] (50% in ethyl acetate) at room temperature. The reaction mixture is stirred for 1-3 hours, diluted with dichloromethane, washed with 1N hydrochloric acid and brine. The organic phases are combined, dried over sodium sulfate and the solvent is concentrated under reduced pressure. The residue is purified by flash chromatography (SiO₂ 60F) to afford the title compound.

### General procedure E: (N-Cbz-deprotection)

To a stirred solution of 1 mmol of "N-Cbz-derivative" in 15 ml of tetrahydrofuran are added 100-200 mg Pd/C 10% and the reaction mixture is hydrogenated at 15-20°C. The reaction mixture is filtered and concentrated under reduced pressure. The residue is purified by flash chromatography (SiO₂ 60F) to afford the title compound.

### General procedure F: (mesylation)

To a stirred solution of 1 mmol of "alcohol" in 10 ml of dichloromethane are added 5 mmol of triethylamine and 2 mmol of methansulfonyl chloride at 0°C. The reaction mixture is allowed to stir for 1 hour, diluted with dichloromethane, washed with 1N hydrochloric acid ,and dried over sodium sulfate. The solvent is concentrated under reduced pressure and the residue is purified by flash chromatography (SiO₂ 60F) to afford the title compound or is directly used in the next step without any further purification.

### General procedure G: (ester formation or amide-formation II)

To a stirred solution of 1.0 mmol of "amine" or "alcohol" in 40 ml of dichloromethane are added 1.5 mmol of "acid" and 0.2 mmol of N,N-dimethylaminopyridine. The reaction mixture is cooled to 0°C and 1.5 mmol of N-ethyl-N'-(3-dimethylaminopropyl)carbodiimid (EDC) is added. The reaction mixture is stirred at 0°C for 3-8 hours, washed with 1M aqueous ammonium chloride solution. The aqueous phase is re-extracted with dichloromethane. The combined organic phases are washed with water, dried over sodium sulfate and the solvent is concentrated under reduced pressure. The residue is purified by flash chromatography (SiO₂ 60F) to afford the title compound.

### General procedure H: (N-Cbz-protection)

To a stirred solution of 1 mmol of "amine" in 30 ml of ethyl acetate and 15 ml saturated aqueous sodium carbonate solution at 0°C is treated with 1.1 mmol benzyl chloroformate. The reaction mixture is allowed to reach room temperature and stirred for 8 hours at room temperature. The phases are separated and the aqueous phase is re-extracted with ethyl acetate. The combined organic phases are dried over sodium sulfate and the solvent is concentrated under reduced pressure. The residue is purified by flash chromatography (SiO₂ 60F) to afford the title compound.

### General procedure I (ester saponification)

To a stirred solution of 1 mmol of "ester" in 3 ml of tetrahydrofuran are added 120 mmol of 3M LiOH solution and the reaction mixture is stirred for 1 hour at room temperature. The mixture is acidified to pH 2 with 2M HCl solution and extracted with ethyl acetate (3X). The combined organic phases are dried over sodium sulfate and the solvent is concentrated under reduced pressure. The residue is the crude title compound.

### General procedure Q (N-Boc-deprotection)

To a stirred solution of 1 mmol "N-Boc-derivative" in 10 ml of dichloromethane at 0°C are added dropwise 15 mmol of trifluoroacetic acid. The reaction mixture is stirred for 2-10 hours at room temperature and then concentrated under reduced pressure. The residue is dissolved in tert-butanol and lyophilized to afford the title compound.

### General procedure R (N-Boc-protection)

To a stirred solution of 1 mmol of "amine" and 1.2 mmol of triethylamine in 12 ml of dichloromethane are added 1.15 mmol of di-tert-butyldicarbonate. The reaction mixture is stirred for 14 hours at room temperature. The mixture is diluted with 20 ml of dichloromethane and washed successively with 10 ml of 0.2N HCl, 10 ml of saturated sodium bicarbonate solution and 10 ml of brine. The organic phase is dried over sodium sulfate and the solvent is concentrated under reduced pressure. The residue is purified by flash chromatography (SiO₂ 60F) to afford the title compound.

### General procedure V: (lactone-amidation)

A mixture of 1 mmol of "lactone", "amine" (5 - 30 equiv.) and 1 mmol of 2-hydroxypyridine is stirred for 2-72 hours at 40-55°C. The reaction mixture is treated with 30 ml of 1M sodium bicarbonate solution and extracted with tert-butyl methyl ether (2X). The combined organic phases are dried over sodium sulfate and the solvent is concentrated under reduced pressure. The residue is purified by flash chromatography (SiO₂ 60F) to afford the title compound.

### General procedure W: (Grignard-reaction)

A solution of 1 mmol of dibutylmagnesium (1M in heptane) in 3.6 ml of tetrahydrofuran is cooled to 0°C and treated dropwise with 1 mmol of butyllithium-solution (1.6M in hexane). The reaction mixture is stirre for 10 minutes at 0°C and a solution of 1 mmol of "arylbromide" or "heteroarylbromide" in 1.4 ml of tetrahydrofuran is added dropwise. The reaction mixture is stirred for 15 minutes at 0°C, then cooled to -78°C and a solution of 1 mmol of 2-[2-azido-2-(4-isopropyl-5-oxo-tetrahydro-furan-2-yl)-ethyl]-3-methyl-butyraldehyd [173154-02-4] in 1.4 ml of tetraahydrofuran, precooled to -78°C, is added dropwise. The reaction mixture is stirred for 1 hour at -78°C, then quenched by addition of 1M ammonium chloride solution. The mixture is extracted with tert-butyl methyl ether (3X). The combined organic phases are washed with brine, dried over sodium sulfate and the solvent is concentrated under reduced pressure. The residue is purified by flash chromatography (SiO₂ 60F) to afford the title compound.

### General procedure X: (alcohol-methoxyacetylation)

To a stirred solution of 1 mmol of "alcohol" in 13.5 ml of toluene at 0°C are added 2.6 mmol of pyridine, 2.4 mmol of methoxyacetylchloride and 0.1 mmol of 4-N,N-dimethylaminopyridine. The cooling bath is removed and the reaction mixture is stirred for 2 hours at room temperature. The reaction mixture is poured onto 0.5M HCl and the organic phase is separated. The aqueous phase is extracted with diethyl ether (3X) and the combined organic phases are washed with brine, dried over sodium sulfate and the solvent is concentrated under reduced pressure. The residue is purified by flash chromatography (SiO₂ 60F) to afford the title compound.

### General procedure Y (hydrogenation)

To a stirred solution of 1 mmol of "substrat" in 25 ml of ethanol and ethanolamine (1 mmol) are added 600 mg Pd/C 10% and the reaction mixture is hydrogenated at room temperature for 2-5 hours. The reaction mixture is filtered, the filtercake is washed with ethanol and the filtrate is concentrated under reduced pressure. The residue is treated with 1M sodium bicarbonate solution and extracted with tert-butyl methyl ether (3X). The combined organic phases are dried over sodium sulfate and the solvent is concentrated under reduced pressure. The residue is purified by flash chromatography (SiO₂ 60F) to afford the title compound.

### General procedure Z (hydrogenation with concomitant N-Boc-protection)

To a stirred solution of 1 mmol of "pyridine derivative" in 20 ml of ethanol are added 3 mmol di-tert-butyl dicarbonate, 3 mmol N,N-diisopropylethylamine and 0.25 mmol Pd/C 10% and the reaction mixture is hydrogenated at room temperature until complete conversion. The reaction mixture is filtered and concentrated under reduced pressure. The residue is purified by flash chromatography (SiO₂ 60F) to afford the title compound.

| | | |
|---|---|---|
| 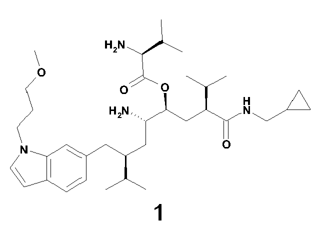 | 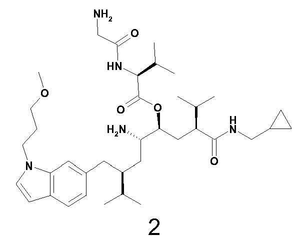 | 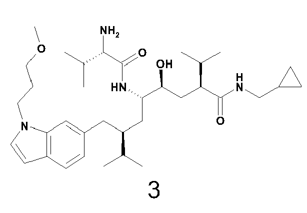 |
| 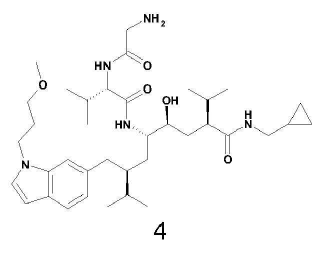 | 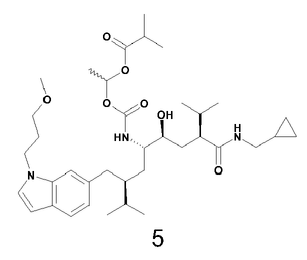 | |
| 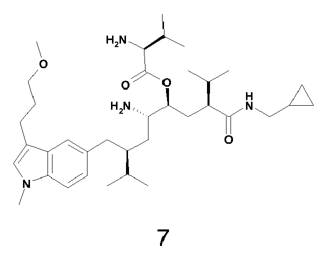 | 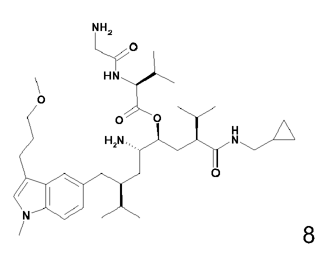 | 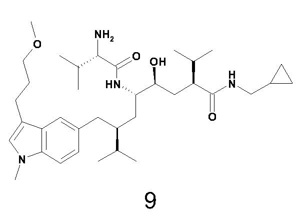 |
| 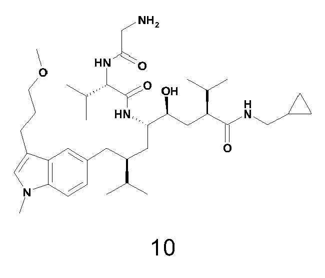 | 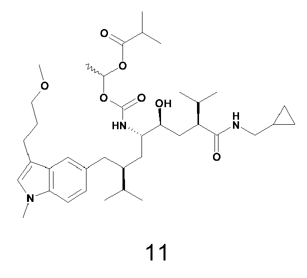 | |
| 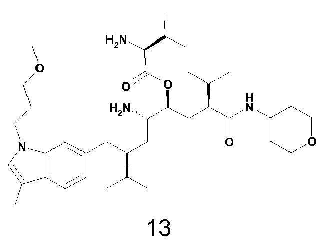 | 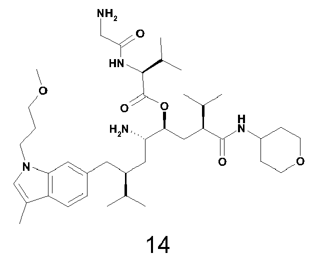 | 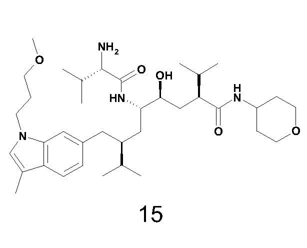 |
| 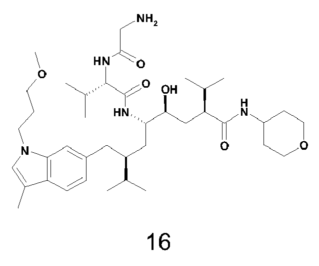 | 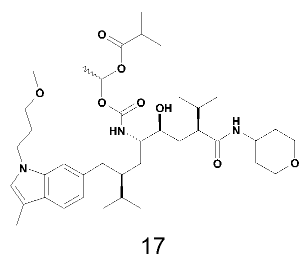 | |
| 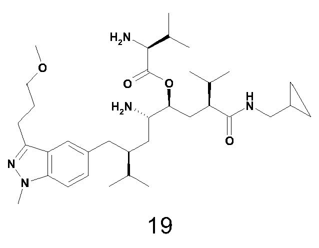 | 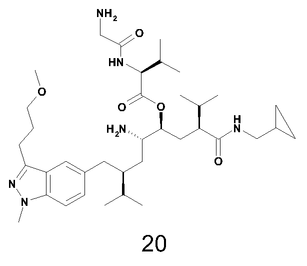 | 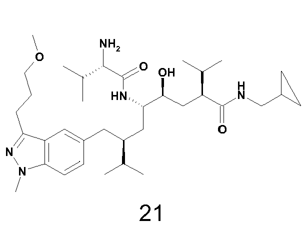 |
| 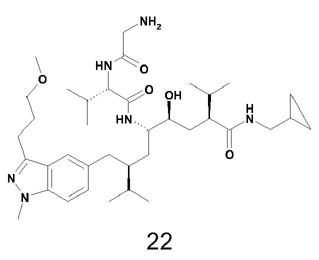 | 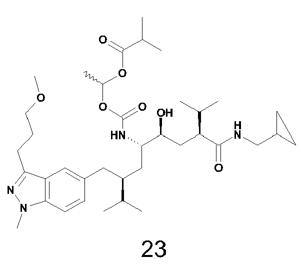 | |
| 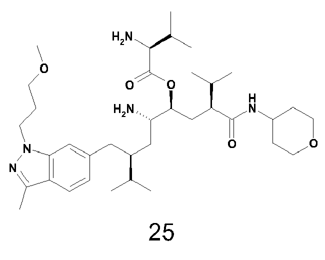 | 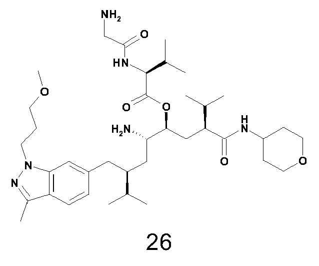 | 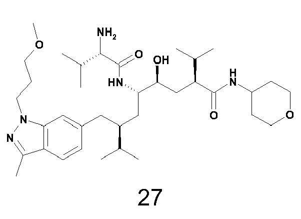 |
| 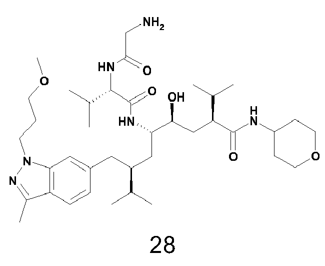 | 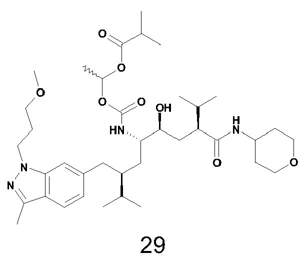 | |
| 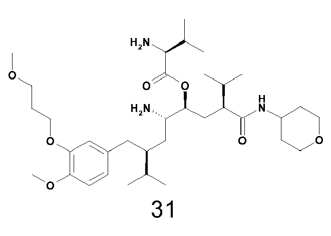 | 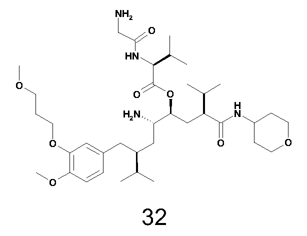 | 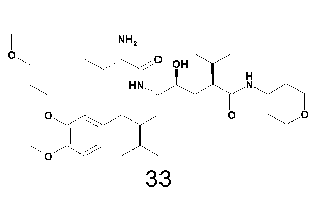 |
| 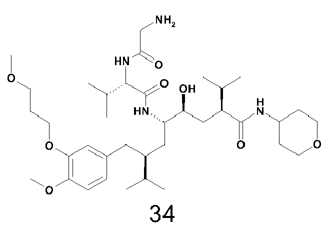 | 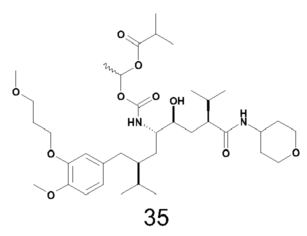 | |
| 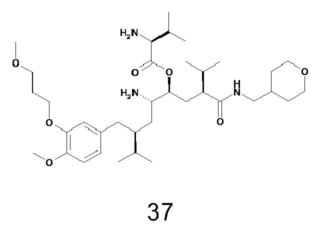 | 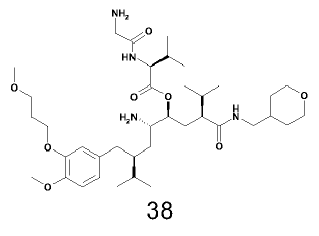 | 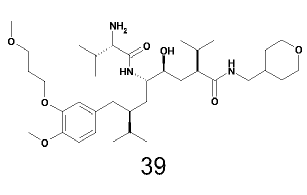 |
| 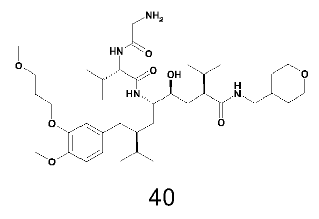 | 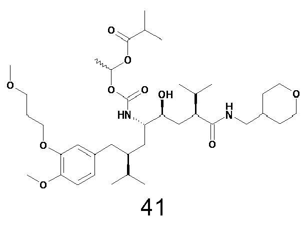 | 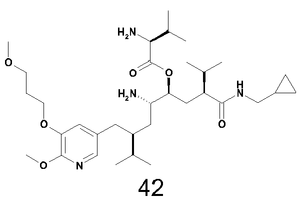 |
| 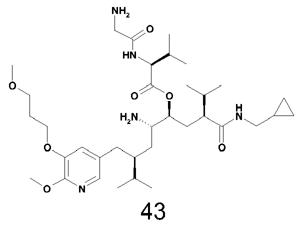 | 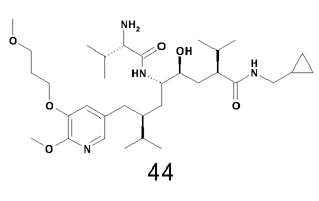 | 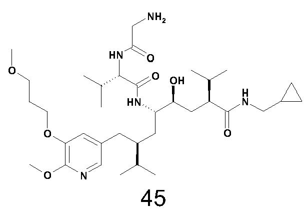 |
| 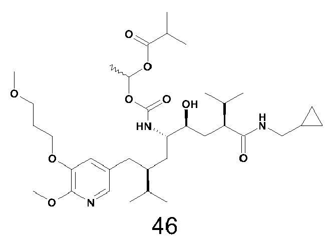 | | 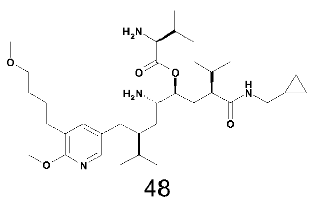 |
| 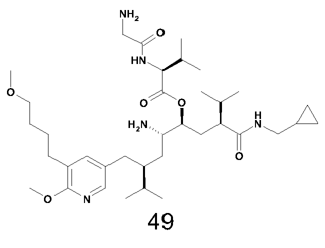 | 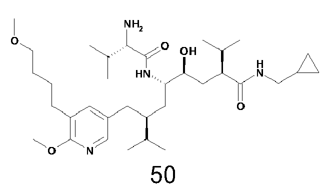 | 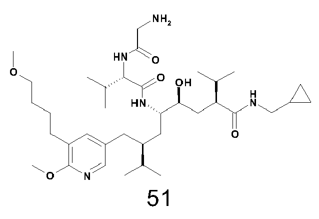 |
| 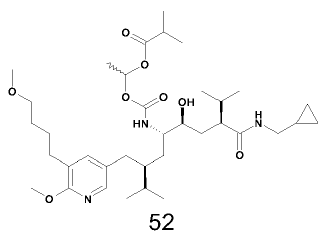 | | |
| 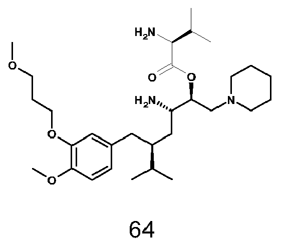 | 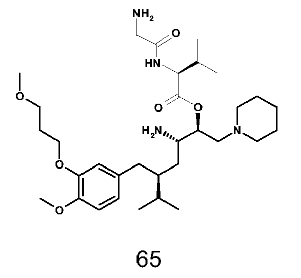 | 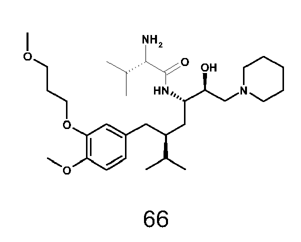 |
| 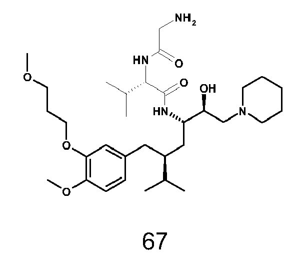 | 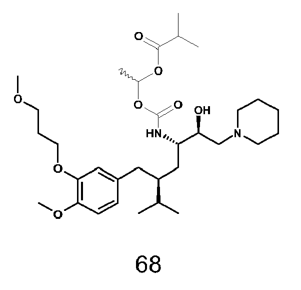 | 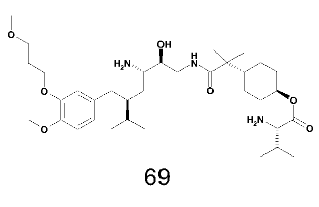 |
| 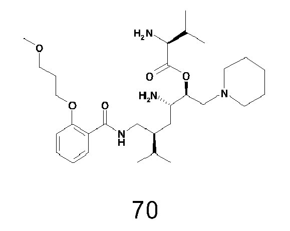 | 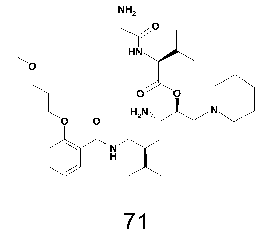 | 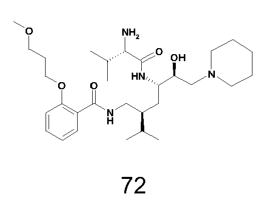 |
| 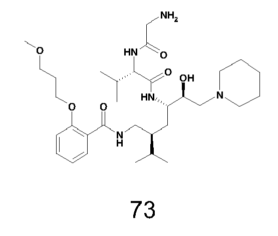 | 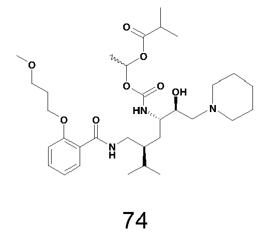 | 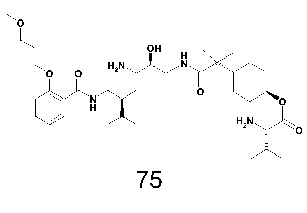 |
| 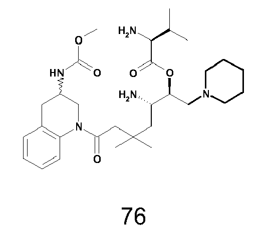 | 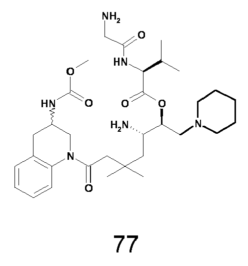 | 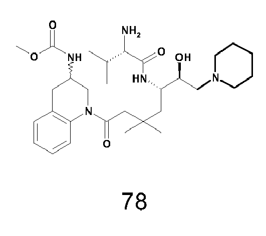 |
| 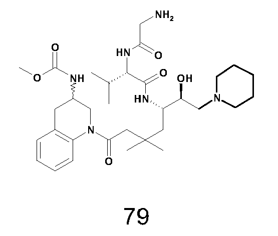 | 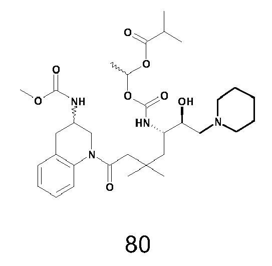 | 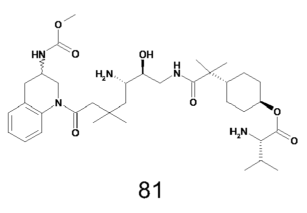 |

| | | |
|---|---|---|
| (*) The below mentioned "heteroarylbromides" are prepared as described in detail in WO 2005/090305 pages 20 to 39 which description is herewith incorporated. | | |

### Example 1

### (S)-2-Amino-3-methyl-butyric acid (1S,2S,4S)-2-amino-1-[(S)-2-(cyclopropylmethyl-carbamoyl)-3-methyl-butyl]-4-[1-(3-methoxy-propyl)-1H-indol-6-ylmethyl]-5-methyl-hexyl ester, bis(trifluoroacetate)

According to general procedure Q, (S)-2-tert-butoxycarbonylamino-3-methyl-butyric acid (1S,2S,4S)-2-tert-butoxycarbonylamino-1-[(S)-2-(cyclopropylmethyl-carbamoyl)-3-methylbutyl]-4-[1-(3-methoxy-propyl)-1H-indol-6-ylmethyl]-5-methyl-hexyl ester is used to afford the title compound, which is identified based on its Rf value.

The starting materials are prepared as follows:
a) (S)-2-tert-Butoxycarbonylamino-3-methyl-butyric acid (1S,2S,4S)-2-tert-butoxycarbonylamino-1-[(S)-2-(cyclopropylmethyl-carbamoyl)-3-methyl-butyl]-4-[1-(3-methoxy-propyl)-1H-indol-6-ylmethyl]-5-methyl-hexyl ester
   According to general procedure G, ((1 S,2S,4S)-4-(cyclopropylmethyl-carbamoyl)-2-hydroxy-1-{(S)-2-[1-(3-methoxy-propyl)-1H-indol-6-ylmethyl]-3-methyl-butyl}-5-methyl-hexyl)-carbamic acid tert-butyl ester and (S)-2-tert-butoxycarbonylamino-3-methyl-butyric acid [13734-41-3] are used to afford the title compound, which is identified based on its Rf value.
b) (1S,2S,4S)-4-(Cyclopropylmethyl-carbamoyl)-2-hydroxy-1-{(S)-2-[1-(3-methoxy-propyl)-1H-indol-6-ylmethyl]-3-methyl-butyl}-5-methyl-hexyl)-carbamic acid tert-butyl ester
   According to general procedure V, {(1S,3S)-1-((S)-4-isopropyl-5-oxo-tetrahydro-furan-2-yl)-3-[1-(3-methoxy-propyl)-1H-indol-6-ylmethyl]-4-methyl-pentyl}-carbamic acid tert-butyl ester and cyclopropylmethylamine [2516-47-4] are used to afford the title compound, which is identified based on its Rf value.
c) {(1S,3S)-1-((S)-4-Isopropyl-5-oxo-tetrahydro-furan-2-yl)-3-[1-(3-methoxy-propyl)-1H-indol-6-ylmethyl-4-methyl-pentyl}-carbamic acid tert-butyl ester
   According to general procedure R, (S)-5-{(1S,3S)-1-amino-3-[1-(3-methoxy-propyl)-1H-indol-6-ylmethyl]-4-methyl-pentyl}-3-isopropyl-dihydro-furan-2-one is used to afford the title compound as a white solid. Rf = 0.31 (EtOAc-heptane 3:1); Rt = 5.87 (gradient I).
d) (S)-5-{(1S,3S)-1-Amino-3-[1-(3-methoxy-propyl)-1H-indol-6-ylmethyl]-4-methyl-pentyl}-3-isopropyl-dihydro-furan-2-one
   According to general procedure Y, methoxy-acetic acid (S)-2-[(S)-2-azido-2-((2S,4S)-4-isopropyl-5-oxo-tetrahydro-furan-2-yl)-ethyl]-1-[1-(3-methoxy-propyl)-1H-indol-6-yl]-3-methylbutyl ester is used to afford the title compound as a colourless yellow oil. Rf = 0.33 (EtOAc-heptane 2:1); Rt = 4.33 (gradient l).
e) Methoxy-acetic acid (S)-2-[(S)-2-azido-2-((2S,4S)-4-isopropyl-5-oxo-tetrahydro-furan-2-yl)-ethyl]-1-[1-(3-methoxy-propyl)-1H-indol-6-yl]-3-methyl-butyl ester
   According to general procedure X, (3S,5S)-5-((1S,3S)-1-azido-3-{hydroxy-[1-(3-methoxypropyl)-1H-indol-6-yl]-methyl}-4-methyl-pentyl)-3-isopropyl-dihydro-furan-2-one is used to afford the title compound as a slightly yellow oil. Rf = 0.24 (EtOAc-heptane 1:1); Rt = 24.04 (gradient II).
f) (3S,5S)-5-((1S,3S)-1-Azido-3-{hydroxy-[1-(3-methoxy-propyl)-1H-indol-6-yl]-methyl}-4-methyl-pentyl)-3-isopropyl-dihydro-furan-2-one
   According to general procedure W, 6-bromo-1-(3-methoxy-propyl)-1H-indole (*) is used to afford the title compound as a slightly yellow oil. Rf = 0.35 (EtOAc-heptane 1:1); Rt = 23.06 (gradient II).

According to the procedure described in example 1, the following compounds are prepared in analogous manner:
- 7: (S)-2-Amino-3-methyl-butyric acid (1S,2S,4S)-2-amino-1-[(S)-2-(cyclopropylmethyl-carbamoyl)-3-methyl-butyl]-4-[3-(3-methoxy-propyl)-1-methyl-1H-indol-5-ylmethyl]-5-methyl-hexyl ester, bis(trifluoroacetate)
starting from 5-bromo-3-(3-methoxy-propyl)-1-methyl-1H-indole (*)
- 13: (S)-2-Amino-3-methyl-butyric acid (1S,2S,4S)-2-amino-4-[1-(3-methoxy-propyl)-3-methyl-1H-indol-6-ylmethyl]-5-methyl-1-[(S)-3-methyl-2-(tetrahydro-pyran-4-ylcarbamoyl)-butyl]-hexyl ester, bis(trifluoroacetate)
starting from 6-bromo-1-(3-methoxy-propyl)-3-methyl-1H-indole (*) and using tetrahydropyran-4-ylamine [38041-19-9] in step b
- 19: (S)-2-Amino-3-methyl-butyric acid (1S,2S,4S)-2-amino-1-[(S)-2-(cyclopropylmethyl-carbamoyl)-3-methyl-butyl]-4-[3-(3-methoxy-propyl)-1-methyl-1H-indazol-5-ylmethyl]-5-methyl-hexyl ester, bis(trifluoroacetate)
starting from 5-bromo-3-(3-methoxy-propyl)-1-methyl-1H-indazole (*)
- 25: (S)-2-Amino-3-methyl-butyric acid (1S,2S,4S)-2-amino-4-[1-(3-methoxy-propyl)-3-methyl-1H-indazol-6-ylmethyl]-5-methyl-1-[(S)-3-methyl-2-(tetrahydro-pyran-4-ylcarbamoyl)-butyl]-hexyl ester, bis(trifluoroacetate)
starting from 6-bromo-1-(3-methoxy-propyl)-3-methyl-1H-indazole (*) and using tetrahydropyran-4-ylamine [38041-19-9] in step b
- 31: (S)-2-Amino-3-methyl-butyric acid (1S,2S,4S)-2-amino-4-[4-methoxy-3-(3-methoxy-propoxy)-benzyl]-5-methyl-1-[(S)-3-methyl-2-(tetrahydro-pyran-4-ylcarbamoyl)-butyl]-hexyl ester, bis(trifluoroacetate)
starting from 4-bromo-1-methoxy-2-(3-methoxy-propoxy)-benzene [173336-76-0] and using tetrahydro-pyran-4-ylamine [38041-19-9] in step b
- 37: (S)-2-Amino-3-methyl-butyric acid (1S,2S,4S)-2-amino-4-[4-methoxy-3-(3-methoxy-propoxy)-benzyl]-5-methyl-1-{(S)-3-methyl-2-[(tetrahydro-pyran-4-ylmethyl)-carbamoyl]-butyl}-hexyl ester, bis(trifluoroacetate)
starting from 4-bromo-1-methoxy-2-(3-methoxy-propoxy)-benzene [173336-76-0] and using C-(tetrahydro-pyran-4-yl)-methylamine [130290-79-8] in step b
- 42: (S)-2-Amino-3-methyl-butyric acid (1S,2S,4S)-2-amino-1-[(S)-2-(cyclopropylmethyl-carbamoyl)-3-methyl-butyl]-4-[6-methoxy-5-(3-methoxy-propoxy)-pyridin-3-ylmethyl]-5-methyl-hexyl ester, bis(trifluoroacetate)
starting from 5-bromo-2-methoxy-3-(3-methoxy-propoxy)-pyridine (*) - the transformation of steps c and d is carried out in one step as indicated in general procedure Z.
- 48: (S)-2-Amino-3-methyl-butyric acid (1S,2S,4S)-2-amino-1-[(S)-2-(cyclopropylmethyl-carbamoyl)-3-methyl-butyl]-4-[6-methoxy-5-(4-methoxy-butyl)-pyridin-3-ylmethyl]-5-methyl-hexyl ester, bis(trifluoroacetate)
starting from 5-bromo-2-methoxy-3-(4-methoxy-butyl)-pyridine (*) - the transformation of steps c and d is carried out in one step as indicated in general procedure Z.

According to the procedure described in example 1 and using (S)-2-(2-tert-butoxycarbonylamino-acetylamino)-3-methyl-butyric acid [28334-73-8] in step a, the following compounds are prepared in analogous manner:
- 2: (S)-2-(2-Amino-acetylamino)-3-methyl-butyric acid (1S,2S,4S)-2-amino-1-[(S)-2-(cyclopropylmethyl-carbamoyl)-3-methyl-butyl]-4-[1-(3-methoxy-propyl)-1H-indol-6-ylmethyl]-5-methyl-hexyl ester, bis(trifluoroacetate)
- 8: (S)-2-(2-Amino-acetylamino)-3-methyl-butyric acid (1S,2S,4S)-2-amino-1-[(S)-2-(cyclopropylmethyl-carbamoyl)-3-methyl-butyl]-4-[3-(3-methoxy-propyl)-1-methyl-1H-indol-5-ylmethyl]-5-methyl-hexyl ester, bis(trifluoroacetate)
starting from 5-bromo-3-(3-methoxy-propyl)-1-methyl-1H-indole (*)
- 14: (S)-2-(2-Amino-acetylamino)-3-methyl-butyric acid (1S,2S,4S)-2-amino-4-[1-(3-methoxy-propyl)-3-methyl-1H-indol-6-ylmethyl]-5-methyl-1-[(S)-3-methyl-2-(tetrahydro-pyran-4-ylcarbamoyl)-butyl]-hexyl ester, bis(trifluoroacetate)
starting from 6-bromo-1-(3-methoxy-propyl)-3-methyl-1H-indole (*) and using tetrahydropyran-4-ylamine [38041-19-9] in step b
- 20: (S)-2-(2-Amino-acetylamino)-3-methyl-butyric acid (1S,2S,4S)-2-amino-1-[(S)-2-(cyclopropylmethyl-carbamoyl)-3-methyl-butyl]-4-[3-(3-methoxy-propyl)-1-methyl-1H-indazol-5-ylmethyl]-5-methyl-hexyl ester, bis(trifluoroacetate)
starting from 5-bromo-3-(3-methoxy-propyl)-1-methyl-1H-indazole (*)
- 26: (S)-2-(2-Amino-acetylamino)-3-methyl-butyric acid (1S,2S,4S)-2-amino-4-[1-(3-methoxy-propyl)-3-methyl-1H-indazol-6-ylmethyl]-5-methyl-1-[(S)-3-methyl-2-(tetrahydro-pyran-4-ylcarbamoyl)-butyl]-hexylester, bis(trifluoroacetate)
starting from 6-bromo-1-(3-methoxy-propyl)-3-methyl-1H-indazole (*) and using tetrahydropyran-4-ylamine [38041-19-9] in step b
- 32: (S)-2-(2-Amino-acetylamino)-3-methyl-butyric acid (1S,2S,4S)-2-amino-4-[4-methoxy-3-(3-methoxy-propoxy)-benzyl]-5-methyl-1-[(S)-3-methyl-2-(tetrahydro-pyran-4-ylcarbamoyl)-butyl]-hexyl ester, bis(trifluoroacetate)
starting from 4-bromo-1-methoxy-2-(3-methoxy-propoxy)-benzene [173336-76-0] and using tetrahydro-pyran-4-ylamine [38041-19-9] in step b
- 38: (S)-2-(2-Amino-acetylamino)-3-methyl-butyric acid (1S,2S,4S)-2-amino-4-[4-methoxy-3-(3-methoxy-propoxy)-benzyl]-5-methyl-1-{(S)-3-methyl-2-[(tetrahydro-pyran-4-ylmethyl)-carbamoyl]-butyl}-hexyl ester, bis(trifluoroacetate)
starting from 4-bromo-1-methoxy-2-(3-methoxy-propoxy)-benzene [173336-76-0] and using C-(tetrahydro-pyran-4-yl)-methylamine [130290-79-8] in step b
- 43: (S)-2-(2-Amino-acetylamino)-3-methyl-butyric acid (1S,2S,4S)-2-amino-1-[(S)-2-(cyclopropylmethyl-carbamoyl)-3-methyl-butyl]-4-[6-methoxy-5-(3-methoxy-propoxy)-pyridin-3-ylmethyl]-5-methyl-hexyl ester, bis(trifluoroacetate)
starting from 5-bromo-2-methoxy-3-(3-methoxy-propoxy)-pyridine (*) - the transformation of steps c and d is carried out in one step as indicated in general procedure Z.
- 49: (S)-2-(2-Amino-acetylamino)-3-methyl-butyric acid (1S,2S,4S)-2-amino-1-[(S)-2-(cyclopropylmethyl-carbamoyl)-3-methyl-butyl]-4-[6-methoxy-5-(4-methoxy-butyl)-pyridin-3-ylmethyl]-5-methyl-hexyl ester, bis(trifluoroacetate)
starting from 5-bromo-2-methoxy-3-(4-methoxy-butyl)-pyridine (*) - the transformation of steps c and d is carried out in one step as indicated in general procedure Z.

### Example 3

### (2S,4S,5S,7S)-5-((S)-2-Amino-3-methyl-butyrylamino)-4-hydroxy-2-isopropyl-7-[1-(3-methoxy-propyl)-1H-indol-6-ylmethyl]-8-methyl-nonanoic acid cyclopropylmethyl-amide, mono(trifluoroacetate)

According to general procedure Q, [(S)-1-((1S,2S,4S)-4-(cyclopropylmethyl-carbamoyl)-2-hydroxy-1-{(S)-2-[1-(3-methoxy-propyl)-1H-indol-6-ylmethyl]-3-methyl-butyl}-5-methyl-hexylcarbamoyl)-2-methyl-propyl]-carbamic acid tert-butyl ester is used to afford the title compound, which is identified based on its Rf value.

The starting materials are prepared as follows:
a) [(S)-1-((1S,2S,4S)-4-(Cyclopropylmethyl-carbamoyl)-2-hydroxy-1-{(S)-2-[1-(3-methoxypropyl)-1H-indol-6-ylmethyl]-3-methyl-butyl}-5-methyl-hexylcarbamoyl)-2-methyl-propyl]-carbamic acid tert-butyl ester
   According to general procedure V, ((S)-1-{(1S,3S)-1-((S)-4-isopropyl-5-oxo-tetrahydro-furan-2-yl)-3-[1-(3-methoxy-propyl)-1H-indol-6-ylmethyl]-4-methyl-pentylcarbamoyl}-2-methylpropyl)-carbamic acid tert-butyl ester and cyclopropylmethylamine [2516-47-4] are used to afford the title compound, which is identified based on its Rf value.
b) ((S)-1-{(1S,3S)-1-((S)-4-)sopropyl-5-oxo-tetrahydro-furan-2-yl)-3-[1-(3-methoxy-propyl)-1H-indol-6-ylmethyll-4-methyl-pentylcarbamoyl)-2-methyl-propyl)-carbamic acid tert-butyl ester
   According to general procedure G, (S)-5-{(1S,3S)-1-amino-3-[1-(3-methoxy-propyl)-1H-indol-6-ylmethyl]-4-methyl-pentyl}-3-isopropyl-dihydro-furan-2-one and (S)-2-tert-butoxycarbonylamino-3-methyl-butyric acid [13734-41-3] is used to afford the title compound, which is identified based on its Rf value.
c) (S)-5-{(1S,3S)-1-Amino-3-[1-(3-methoxy-propyl)-1H-indol-6-ylmethyl]-4-methyl-pentyl}-3-isopropyl-dihydro-furan-2-one
   According to general procedure Y, methoxy-acetic acid (S)-2-[(S)-2-azido-2-((2S,4S)-4-isopropyl-5-oxo-tetrahydro-furan-2-yl)-ethyl]-1-[1-(3-methoxy-propyl)-1H-indol-6-yl]-3-methylbutyl ester is used to afford the title compound as a colourless yellow oil. Rf = 0.33 (EtOAc-heptane 2:1); Rt = 4.33 (gradient I).
d) Methoxy-acetic acid (S)-2-[(S)-2-azido-2-((2S,4S)-4-isopropyl-5-oxo-tetrahydro-furan-2-yl)-ethyl]-1-[1-(3-methoxy-propyl)-1H-indol-6-yl]-3-methyl-butyl ester
   According to general procedure X, (3S,5S)-5-((1S,3S)-1-azido-3-{hydroxy-[1-(3-methoxypropyl)-1H-indol-6-yl]-methyl}-4-methyl-pentyl)-3-isopropyl-dihydro-furan-2-one is used to afford the title compound as a slightly yellow oil. Rf = 0.24 (EtOAc-heptane 1:1); Rt = 24.04 (gradient II).
e) (3S,5S)-5-((1S,3S)-1-Azido-3-{hydroxy-[1-(3-methoxy-propyl)-1H-indol-6-yl]-methyl}-4-methyl-pentyl)-3-isopropyl-dihydro-furan-2-one
   According to general procedure W, 6-bromo-1-(3-methoxy-propyl)-1H-indole (*) is used to afford the title compound as a slightly yellow oil. Rf = 0.35 (EtOAc-heptane 1:1); Rt = 23.06 (gradient II).

According to the procedure described in example 3, the following compounds are prepared in analogous manner:
- 9: (2S,4S,5S,7S)-5-((S)-2-Amino-3-methyl-butyrylamino)-4-hydroxy-2-isopropyl-7-[3-(3-methoxy-propyl)-1-methyl-1H-indol-5-ylmethyl]-8-methyl-nonanoic acid cyclopropylmethyl-amide, mono(trifluoroacetate)
starting from 5-bromo-3-(3-methoxy-propyl)-1-methyl-1H-indole (*)
- 15: (2S,4S,5S,7S)-5-((S)-2-Amino-3-methyl-butyrylamino)-4-hydroxy-2-isopropyl-7-[1-(3-methoxy-propyl)-3-methyl-1H-indol-6-ylmethyl]-8-methyl-nonanoic acid (tetrahydropyran-4-yl)-amide, mono(trifluoroacetate)
starting from 6-bromo-1-(3-methoxy-propyl)-3-methyl-1H-indole (*) and using tetrahydropyran-4-ylamine [38041-19-9] in step a
- 21: (2S,4S,5S,7S)-5-((S)-2-Amino-3-methyl-butyrylamino)-4-hydroxy-2-isopropyl-7-[3-(3-methoxy-propyl)-1-methyl-1H-indazol-5-ylmethyl]-8-methyl-nonanoic acid cyclopropylmethyl-amide, mono(trifluoroacetate)
starting from 5-bromo-3-(3-methoxy-propyl)-1-methyl-1H-indazole (*)
- 27: (2S,4S,5S,7S)-5-((S)-2-Amino-3-methyl-butyrylamino)-4-hydroxy-2-isopropyl-7-[1-(3-methoxy-propyl)-3-methyl-1H-indazol-6-ylmethyl]-8-methyl-nonanoic acid (tetrahydropyran-4-yl)-amide, mono(trifluoroacetate)
starting from 6-bromo-1-(3-methoxy-propyl)-3-methyl-1H-indazole (*) and using tetrahydropyran-4-ylamine [38041-19-9] in step a
- 33: (2S,4S,5S,7S)-5-((S)-2-Amino-3-methyl-butyrylamino)-4-hydroxy-2-isopropyl-7-[4-methoxy-3-(3-methoxy-propoxy)-benzyl]-8-methyl-nonanoic acid (tetrahydro-pyran-4-yl)-amide, mono(trifluoroacetate)
starting from 4-bromo-1-methoxy-2-(3-methoxy-propoxy)-benzene [173336-76-0] and using tetrahydro-pyran-4-ylamine [38041-19-9] in step a
- 39: (2S,4S,5S,7S)-5-((S)-2-Amino-3-methyl-butyrylamino)-4-hydroxy-2-isopropyl-7-[4-methoxy-3-(3-methoxy-propoxy)-benzyl]-8-methyl-nonanoic acid (tetrahydro-pyran-4-ylmethyl)-amide, mono(trifluoroacetate)
starting from 4-bromo-1-methoxy-2-(3-methoxy-propoxy)-benzene [173336-76-0] and using C-(tetrahydro-pyran-4-yl)-methylamine [130290-79-8] in step a
- 44: (2S,4S,5S,7S)-5-((S)-2-Amino-3-methyl-butyrylamino)-4-hydroxy-2-isopropyl-7-[6-methoxy-5-(3-methoxy-propoxy)-pyridin-3-ylmethyl]-8-methyl-nonanoic acid cyclopropylmethyl-amide, mono(trifluoroacetate)
starting from 5-bromo-2-methoxy-3-(3-methoxy-propoxy)-pyridine (*) - the transformation of step c is carried out in 2 steps following general procedure Z and general procedure Q.
- 50: (2S,4S,5S,7S)-5-((S)-2-Amino-3-methyl-butyrylamino)-4-hydroxy-2-isopropyl-7-[6-methoxy-5-(4-methoxy-butyl)-pyridin-3-ylmethyl]-8-methyl-nonanoic acid cyclopropylmethyl-amide, mono(trifluoroacetate)
starting from 5-bromo-2-methoxy-3-(4-methoxy-butyl)-pyridine (*) - the transformation of step c is carried out in 2 steps following general procedure Z and general procedure Q.

According to the procedure described in example 3 and using (S)-2-(2-tert-butoxycarbonylamino-acetylamino)-3-methyl-butyric acid [28334-73-8] in step b, the following compounds are prepared in analogous manner:
- 4: (2S,4S,5S,7S)-5-[(S)-2-(2-Amino-acetylamino)-3-methyl-butyrylamino]-4-hydroxy-2-isopropyl-7-[1-(3-methoxy-propyl)-1H-indol-6-ylmethyl]-8-methyl-nonanoic acid cyclopropylmethyl-amide, mono(trifluoroacetate)
- 10: (2S,4S,5S,7S)-5-[(S)-2-(2-Amino-acetylamino)-3-methyl-butyrylamino]-4-hydroxy-2-isopropyl-7-[3-(3-methoxy-propyl)-1-methyl-1H-indol-5-ylmethyl]-8-methyl-nonanoic acid cyclopropylmethyl-amide, mono(trifluoroacetate)
starting from 5-bromo-3-(3-methoxy-propyl)-1-methyl-1H-indole (*)
- 16: (2S,4S,5S,7S)-5-[(S)-2-(2-Amino-acetylamino)-3-methyl-butyrylamino]-4-hydroxy-2-isopropyl-7-[1-(3-methoxy-propyl)-3-methyl-1H-indol-6-ylmethyl]-8-methyl-nonanoic acid (tetrahydro-pyran-4-yl)-amide, mono(trifluoroacetate)
starting from 6-bromo-1-(3-methoxy-propyl)-3-methyl-1H-indole (*) and using tetrahydropyran-4-ylamine [38041-19-9] in step a
- 22: (2S,4S,5S,7S)-5-[(S)-2-(2-Amino-acetylamino)-3-methyl-butyrylamino]-4-hydroxy-2-isopropyl-7-[3-(3-methoxy-propyl)-1-methyl-1H-indazol-5-ylmethyl]-8-methyl-nonanoic acid cyclopropylmethyl-amide, mono(trifluoroacetate)
starting from 5-bromo-3-(3-methoxy-propyl)-1-methyl-1H-indazole (*)
- 28: (2S,4S,5S,7S)-5-[(S)-2-(2-Amino-acetylamino)-3-methyl-butyrylamino]-4-hydroxy-2-isopropyl-7-[1-(3-methoxy-propyl)-3-methyl-1H-indazol-6-ylmethyl]-8-methyl-nonanoic acid (tetrahydro-pyran-4-yl)-amide, mono(trifluoroacetate)
starting from 6-bromo-1-(3-methoxy-propyl)-3-methyl-1H-indazole (*) and using tetrahydropyran-4-ylamine [38041-19-9] in step a
- 34: (2S,4S,5S,7S)-5-[(S)-2-(2-Amino-acetylamino)-3-methyl-butyrylamino]-4-hydroxy-2-isopropyl-7-[4-methoxy-3-(3-methoxy-propoxy)-benzyl]-8-methyl-nonanoic acid (tetrahydro-pyran-4-yl)-amide, mono(trifluoroacetate)
starting from 4-bromo-1-methoxy-2-(3-methoxy-propoxy)-benzene [173336-76-0] and using tetrahydro-pyran-4-ylamine [38041-19-9] in step a
- 40: (2S,4S,5S,7S)-5-[(S)-2-(2-Amino-acetylamino)-3-methyl-butyrylamino]-4-hydroxy-2-isopropyl-7-[4-methoxy-3-(3-methoxy-propoxy)-benzyl]-8-methyl-nonanoic acid (tetrahydro-pyran-4-ylmethyl)-amide, mono(trifluoroacetate)
starting from 4-bromo-1-methoxy-2-(3-methoxy-propoxy)-benzene [173336-76-0] and using C-(tetrahydro-pyran-4-yl)-methylamine [130290-79-8] in step a
- 45: (2S,4S,5S,7S)-5-[(S)-2-(2-Amino-acetylamino)-3-methyl-butyrylamino]-4-hydroxy-2-isopropyl-7-[6-methoxy-5-(3-methoxy-propoxy)-pyridin-3-ylmethyl]-8-methyl-nonanoic acid cyclopropylmethyl-amide, mono(trifluoroacetate)
starting from 5-bromo-2-methoxy-3-(3-methoxy-propoxy)-pyridine (*) - the transformation of step c is carried out in 2 steps following general procedure Z and general procedure Q.
- 51: (2S,4S,5S,7S)-5-[(S)-2-(2-Amino-acetylamino)-3-methyl-butyrylamiono]-4-hydroxy-2-isopropyl-7-[6-methoxy-5-(4-methoxy-butyl)-pyridin-3-ylmethyl]-8-methyl-nonanoic acid cyclopropylmethyl-amide, mono(trifluoroacetate)
starting from 5-bromo-2-methoxy-3-(4-methoxy-butyl)-pyridine (*) - the transformation of step c is carried out in 2 steps following general procedure Z and general procedure Q.

### Example 5

### Isobutyric acid 1-((1S,2S,4S)-4-(cyclopropylmethyl-carbamoyl)-2-hydroxy-1-{(S)-2-[1-(3-methoxy-propyl)-1H-indol-6-ylmethyl]-3-methyl-butyl}-5-methyl-hexylcarbamoyloxy)-ethyl ester (diastereomeric mixture)

To a stirred solution of 1 mmol of ((1S,2S,4S)-4-(cyclopropylmethyl-carbamoyl)-2-hydroxy-1-{(S)-2-[1-(3-methoxy-propyl)-1H-indol-6-ylmethyl]-3-methyl-butyl}-5-methyl-hexyl)-carbamic acid 1-chloro-ethyl ester (diastereomeric mixture) in 30 ml of N,N-dimethylformamide is added 5 mmol caesium carbonate, followed by 2 mmol of isobutyric acid [79-31-2]. The reaction mixture is stirred for 12 hours at room temperature. The reaction mixture is diluted with water and extracted with tert-butyl methyl ether (4X). The combined organic phases are washed with brine, dried over sodium sulfate and the solvent is concentrated under reduced pressure. The residue is purified by flash chromatography (SiO₂60F) to afford the title compound, which is identified based on its Rf value.

The starting materials are prepared as follows:
a) ((1S,2S,4S)-4-(Cyclopropylmethyl-carbamoyl)-2-hydroxy-1-{(S)-2-[1-(3-methoxy-propyl)-1H-indol-6-ylmethyl]-3-methyl-butyl}-5-methyl-hexyl)-carbamic acid 1-chloro-ethyl ester (diastereomeric mixture)
   To a stirred solution of 1 mmol of (2S,4S,5S,7S)-5-amino-4-hydroxy-2-isopropyl-7-[1-(3-methoxy-propyl)-1H-indol-6-ylmethyl]-8-methyl-nonanoic acid cyclopropylmethyl-amide in 20 ml of dichloromethane is added 3 mmol triethylamine. The solution is cooled to 0°C and 1.1 mmol of 1-chloroethyl chloroformate [50893-53-3] is added. The reaction mixture is stirred for 5 minutes at 0°C, then for 3 hours at room temperature. The reaction mixture is diluted with dichloromethane and washed with 1M hydrochloric acid. The aqueous phase is re-extracted with dichloromethane (1X). The combined organic phases are washed with 1M aqueous sodium bicarbonate solution, dried over sodium sulfate and the solvent is concentrated under reduced pressure. The residue is purified by flash chromatography (SiO₂ 60F) to afford the title compound, which is identified based on its Rf value.
b) (2S,4S,5S,7S)-5-Amino-4-hydroxy-2-isopropyl-7-[1-(3-methoxy-propyl)-1H-indol-6-ylmethyl]-8-methyl-nonanoic acid cyclopropylmethyl-amide
   According to general procedure Q, (1S,2S,4S)-4-(cyclopropylmethyl-carbamoyl)-2-hydroxy-1-{(S)-2-[1-(3-methoxy-propyl)-1H-indol-6-ylmethyl]-3-methyl-butyl}-5-methyl-hexyl)-carbamic acid tert-butyl ester is used to afford the title compound as a yellow oil. Rf = 0.37 (dichloromethane-methanol-25% ammonia conc. 200:20:1); Rt = 4.25 (gradient I).
c) (1S,2S,4S)-4-(Cyclopropylmethyl-carbamoyl)-2-hydroxy-1-{(S)-2-[1-(3-methoxy-propyl)-1H-indol-6-ylmethyl]-3-methyl-butyl}-5-methyl-hexyl)-carbamic acid tert-butyl ester
   According to general procedure V, {(1S,3S)-1-((S)-4-isopropyl-5-oxo-tetrahydro-furan-2-yl)-3-[1-(3-methoxy-propyl)-1H-indol-6-ylmethyl]-4-methyl-pentyl}-carbamic acid tert-butyl ester and cyclopropylmethylamine [2516-47-4] are used to afford the title compound, which is identified based on its Rf value.
d) {(1S,3S)-1-((2S,4S)-4-Isopropyl-5-oxo-tetrahydro-furan-2-yl)-3-[1-(3-methoxy-propyl)-1H-indol-6-ylmethyl]-4-methyl-pentyl}-carbamic acid tert-butyl ester
   According to general procedure R, (S)-5-{(1S,3S)-1-amino-3-[1-(3-methoxy-propyl)-1 H-indol-6-ylmethyl]-4-methyl-pentyll-3-isopropyl-dihydro-furan-2-one is used to afford the title compound as a white solid. Rf = 0.31 (EtOAc-heptane 3:1); Rt = 5.87 (gradient I).
e) (3S,5S)-5-{(1S,3S)-1-Amino-3-[1-(3-methoxy-propyl)-1H-indol-6-ylmethyl]-4-methylpentyl}-3-isopropyl-dihydro-furan-2-one
   According to general procedure Y, methoxy-acetic acid (S)-2-[(S)-2-azido-2-((2S,4S)-4-isopropyl-5-oxo-tetrahydro-furan-2-yl)-ethyl]-1-[1-(3-methoxy-propyl)-1H-indol-6-yl]-3-methylbutyl ester is used to afford the title compound as a colourless yellow oil. Rf = 0.33 (EtOAc-heptane 2:1); Rt = 4.33 (gradient I).
f) Methoxy-acetic acid (S)-2-f(S)-2-azido-2-((2S,4S)-4-isopropyl-5-oxo-tetrahydro-furan-2-yl)-ethyll-1-[1-(3-methoxy-propyl)-1H-indol-6-yl]-3-methyl-butyl ester
   According to general procedure X, (3S,5S)-5-((1S,3S)-1-azido-3-{hydroxy-[1-(3-methoxypropyl)-1H-indol-6-yl]-methyl}-4-methyl-pentyl)-3-isopropyl-dihydro-furan-2-one is used to afford the title compound as a slightly yellow oil. Rf = 0.24 (EtOAc-heptane 1:1); Rt = 24.04 (gradient II).
g) (3S,5S)-5-((1S,3S)-1-Azido-3-{hydroxy-[1-(3-methoxy-propyl)-1H-indol-6-yl]-methyl}-4-methyl-pentyl)-3-isopropyl-dihydro-furan-2-one
   According to general procedure W, 6-bromo-1-(3-methoxy-propyl)-1H-indole (*) is used to afford the title compound as a slightly yellow oil. Rf = 0.35 (EtOAc-heptane 1:1); Rt = 23.06 (gradient II).

According to the procedure described in example 5, the following compounds are prepared in analogous manner:
- 11: Isobutyric acid 1-((1S,2S,4S)-4-(cyclopropylmethyl-carbamoyl)-2-hydroxy-1-{(S)-2-[3-(3-methoxy-propyl)-1-methyl-1H-indol-5-ylmethyl]-3-methyl]-butyl}-5-methyl-hexylcarbamoyloxy)-ethyl ester (diastereomeric mixture)
starting from 5-bromo-3-(3-methoxy-propyl)-1-methyl-1H-indole (*)
- 17: Isobutyric acid 1-[(1S,2S,4S)-2-hydroxy-1-{(S)-2-[1-(3-methoxy-propyl)-3-methy-1H-indol-6-ylmethyl]-3-methyl-butyl}-5-methyl-4-(tetrahydro-pyran-4-ylcarbamoyl)-hexylcarbamoyloxy]-ethyl ester (diastereomeric mixture)
starting from 6-bromo-1-(3-methoxy-propyl)-3-methyl-1H-indole (*) and using tetrahydropyran-4-ylamine [38041-19-9] in step c
- 23: Isobutyric acid 1-((1S,2S,4S)-4-(cyclopropylmethyl-carbamoyl)-2-hydroxy-1-{(S)-2-[3-(3-methoxy-propyl)-1-methyl-1H-indazol-5-ylmethyl]-3-methyl-butyl}-5-methyl-hexylcarbamoyloxy)-ethyl ester (diastereomeric mixture)
starting from 5-bromo-3-(3-methoxy-propyl)-1-methyl-1H-indazole (*)
- 29: Isobutyric acid 1-[(1S,2S,4S)-2-hydroxy-1-(S)-2-[1-(3-methoxy-propyl)-3-methyl-1H-indazol-6-ylmethyl]-3-methyl-butyl}-5-methyl-4-(tetrahydro-pyran-4-ylcarbamoyl)-hexylcarbamoyloxy]-ethyl ester (diastereomeric mixture)
starting from 6-bromo-1-(3-methoxy-propyl)-3-methyl-1H-indazole (*) and using tetrahydropyran-4-ylamine [38041-19-9] in step c
- 35: Isobutyric acid 1-[(1S,2S,4S)-2-hydroxy-1-{(S)-2-[4-methoxy-3-(3-methoxy-propoxy)-benzyl]-3-methyl-butyl}-5-methyl-4-(tetrahydro-pyran-4-ylcarbamoyl)-hexylcarbamoyloxyl-ethyl ester (diastereomeric mixture)
starting from 4-bromo-1-methoxy-2-(3-methoxy-propoxy)-benzene [173336-76-0] and using tetrahydro-pyran-4-ylamine [38041-19-9] in step c
- 41: Isobutyric acid 1-{(1S,2S,4S)-2-hydroxy-1-{(S)-2-[4-methoxy-3-(3-methoxy-propoxy)-benzyl]-3-methyl-butyl}-5-methyl-4-[(tetrahydro-pyran-4-ylmethyl)-carbamoyl]-hexylcarbamoyloxy}-ethyl ester (diastereomeric mixture)
starting from 4-bromo-1-methoxy-2-(3-methoxy-propoxy)-benzene [173336-76-0] and using C-(tetrahydro-pyran-4-yl)-methylamine [130290-79-8] in step c
- 46: Isobutyric acid 1-((1S,2S,4S)-4-(cyclopropylmethyl-carbamoyl)-2-hydroxy-1-{(S)-2-[6-methoxy-5-(3-methoxy-propoxy)-pyridin-3-ylmethyl]-3-methyl-butyl}-5-methyl-hexylcarbamoyloxy)-ethyl ester (diastereomeric mixture)
starting from 5-bromo-2-methoxy-3-(3-methoxy-propoxy)-pyridine (*) - the transformation of steps d and e is carried out in one step as indicated in general procedure Z.
- 52: Isobutyric acid 1-((1S,2S,4S)-4-(cyclopropylmethyl-carbamoyl)-2-hydroxy-1-{(S)-2-[6-methoxy-5-(4-methoxy-butyl)-pyridin-3-ylmethyl]-3-methyl-butyl}-5-methyl-hexylcarbamoyloxy)-ethyl ester (diastereomeric mixture)
starting from 5-bromo-2-methoxy-3-(4-methoxy-butyl)-pyridine (*) - the transformation of steps d and e is carried out in one step as indicated in general procedure Z.

### Example 64

### (S)-2-Amino-3-methyl-butyric acid (1S,2S,4S)-2-amino-4-[4-methoxy-3-(3-methoxy-propoxy)-benzyl]-5-methyl-1-piperidin-1-ylmethyl-hexyl ester, tris(trifluoroacetate)

According to general procedure Q, (S)-2-tert-butoxycarbonylamino-3-methyl-butyric acid (1S,2S,4S)-2-tert-butoxycarbonylamino-4-[4-methoxy-3-(3-methoxy-propoxy)-benzyl]-5-methyl-1-piperidin-1-ylmethyl-hexyl ester is used to afford the title compound, which is identified based on its Rf value.

The starting materials are prepared as follows:
a) (S)-2-tert-Butoxycarbonylamino-3-methyl-butyric acid (1S,2S,4S)-2-tert-butoxycarbonylamino-4-[4-methoxy-3-(3-methoxy-propoxy)-benzyl]-5-methyl-1-piperidin-1-ylmethyl-hexyl ester
   According to general procedure G, {(1S,3S)-1-((S)-1-hydroxy-2-piperidin-1-yl-ethyl)-3-[4-methoxy-3-(3-methoxy-propoxy)-benzyl]-4-methyl-pentyl}-carbamic acid tert-butyl ester and (S)-2-tert-butoxycarbonylamino-3-methyl-butyric acid [13734-41-3] are used to afford the title compound, which is identified based on its Rf value.
b) {(1S,3S)-1-((S)-1-Hydroxy-2-piperidin-1-yl-ethyl)-3-[4-methoxy-3-(3-methoxy-propoxy)-benzyl]-4-methyl-pentyl}-carbamic acid tert-butyl ester
   According to general procedure R, (2S,3S,5S)-3-amino-5-[4-methoxy-3-(3-methoxy-propoxy)-benzyl]-6-methyl-1-piperidin-1-yl-heptan-2-ol [861922-24-9] is used to afford the title compound, which is identified based on its Rf value.

According to the procedure described in example 64, the following compounds are prepared in analogous manner:
- 70: (S)-2-Amino-3-methyl-butyric acid (1S,2S,4S)-2-amino-4-{[2-(3-methoxy-propoxy)-benzoylamino]-methyl}-5-methyl-1-piperidin-1-ylmethyl-hexyl ester, tris(trifluoroacetate)
starting from N-((2S,4S,5S)-4-amino-5-hydroxy-2-isopropyl-6-piperidin-1-yl-hexyl)-2-(3-methoxy-propoxy)-benzamide [861772-91-0]
- 76: (S)-2-Amino-3-methyl-butyric acid (1S,2S)-2-amino-6-(3-methoxycarbonylamino-3,4-dihydro-2H-quinolin-1-yl)-4,4-dimethyl-6-oxo-1-piperidin-1-ylmethyl-hexyl ester, tris(trifluoroacetate)
starting from [1-((5S,6S)-5-amino-6-hydroxy-3,3-dimethyl-7-piperidin-1-yl-heptanoyl)-1,2,3,4-tetrahydro-quinolin-3-yl]-carbamic acid methyl ester [861444-61-3]

According to the procedure described in example 64 and using (S)-2-(2-tert-butoxycarbonylamino-acetylamino)-3-methyl-butyric acid [28334-73-8] in step a, the following compounds are prepared in analogous manner:
- 65: (S)-2-(2-Amino-acetylamino)-3-methyl-butyric acid (1S,2S,4S)-2-amino-4-[4-methoxy-3-(3-methoxy-propoxy)-benzyl]-5-methyl-1-piperidin-1-ylmethyl-hexyl ester, tris(trifluoroacetate)
- 71: (S)-2-(2-Amino-acetylamino)-3-methyl-butyric acid (1S,2S,4S)-2-amino-4-{[2-(3-methoxy-propoxy)-benzoylamino]-methyl}-5-methyl-1-piperidin-1-ylmethyl-hexyl ester, tris(trifluoroacetate)
starting from N-((2S,4S,5S)-4-amino-5-hydroxy-2-isopropyl-6-piperidin-1-yl-hexyl)-2-(3-methoxy-propoxy)-benzamide [861772-91-0]
- 77: (S)-2-(2-Amino-acetylamino)-3-methyl-butyric acid (1S,2S)-2-amino-6-(3-methoxycarbonylamino-3,4-dihydro-2H-quinolin-1-yl)-4,4-dimethyl-6-oxo-1-piperidin-1-ylmethyl-hexyl ester, tris(trifluoroacetate)
starting from [1-((5S,6S)-5-amino-6-hydroxy-3,3-dimethyl-7-piperidin-1-yl-heptanoyl)-1,2,3,4-tetrahydro-quinolin-3-yl]-carbamic acid methyl ester [861444-61-3]

### Example 66

### (S)-2-Amino-N-{(1S,3S)-1-((S)-1-hydroxy-2-piperidin-1-yl-ethyl)-3-[4-methoxy-3-(3-methoxy-propoxy)-benzyl]-4-methyl-pentyl}-3-methyl-butyramide, bis(trifluoroacetate)

According to general procedure Q, ((S)-1-{(1S,3S)-1-((S)-1-hydroxy-2-piperidin-1-yl-ethyl)-3-[4-methoxy-3-(3-methoxy-propoxy)-benzyl]-4-methyl-pentylcarbamoyl}-2-methyl-propyl)-carbamic acid tert-butyl ester is used to afford the title compound, which is identified based on its Rf value.

The starting material is prepared as follows:
a) ((S)-1-{(1S,3S)-1-((S)-1-Hydroxy-2-piperidin-1-yl-ethyl)-3-[4-methoxy-3-(3-methoxy-propoxy)-benzyl]-4-methyl-pentylcarbamoyl}-2-methyl-propyl)-carbamic acid tert-butyl ester
   According to general procedure G, (2S,3S,5S)-3-amino-5-[4-methoxy-3-(3-methoxy-propoxy)-benzyl]-6-methyl-1-piperidin-1-yl-heptan-2-ol [861922-24-9] and (S)-2-tert-butoxycarbonylamino-3-methyl-butyric acid [13734-41-3] are used to afford the title compound, which is identified based on its Rf value.

According to the procedure described in example 66, the following compounds are prepared in analogous manner:
- 72: N-[(2S,4S,5S)-4-((S)-2-Amino-3-methyl-butyrylamino)-5-hydroxy-2-isopropyl-6-piperidin-1-yl-hexyl]-2-(3-methoxy-propoxy)-benzamide, bis(trifluoroacetate)
starting from N-((2S,4S,5S)-4-amino-5-hydroxy-2-isopropyl-6-piperidin-1-yl-hexyl)-2-(3-methoxy-propoxy)-benzamide [861772-91-0]
- 78: {1-[(5S,6S)-5-((S)-2-Amino-3-methyl-butyrylamino)-6-hydroxy-3,3-dimethyl-7-piperidin-1-yl-heptanoyl]-1,2,3,4-tetrahydro-quinolin-3-yl}-carbamic acid methyl ester, bis(trifluoroacetate)
starting from [1-((5S,6S)-5-amino-6-hydroxy-3,3-dimethyl-7-piperidin-1-yl-heptanoyl)-1,2,3,4-tetrahydro-quinolin-3-yl]-carbamic acid methyl ester [861444-61-3]

According to the procedure described in example 66 and using (S)-2-(2-tert-butoxycarbonylamino-acetylamino)-3-methyl-butyric acid [28334-73-8] in step a, the following compounds are prepared in analogous manner:
- 67: (S)-2-(2-Amino-acetylamino)-N-{(1S,3S)-1-((S)-1-hydroxy-2-piperidin-1-yl-ethyl)-3-[4-methoxy-3-(3-methoxy-propoxy)-benzyl]-4-methyl-pentyl}-3-methyl-butyramide, bis(trifluoroacetate)
- 73: N-{(2S,4S,5S)-4-[(S)-2-(2-Amino-acetylamino)-3-methyl-butyrylamino]-5-hydroxy-2-isopropyl-6-piperidin-1-yl-hexyl}-2-(3-methoxy-propoxy)-benzamide, bis(trifluoroacetate)
starting from N-((2S,4S,5S)-4-amino-5-hydroxy-2-isopropyl-6-piperidin-1-yl-hexyl)-2-(3-methoxy-propoxy)-benzamide [861772-91-0]
- 79: (1-{(5S,6S)-5-[(S)-2-(2-Amino-acetylamino)-3-methyl-butyrylamino]-6-hydroxy-3,3-dimethyl-7-piperidin-1-yl-heptanoyl}-1,2,3,4-tetrahydro-auinolin-3-yl)-carbamic acid methyl ester, bis(trifluoroacetate)
starting from [1-((5S,6S)-5-amino-6-hydroxy-3,3-dimethyl-7-piperidin-1-yl-heptanoyl)-1,2,3,4-tetrahydro-quinolin-3-yl]-carbamic acid methyl ester [861444-61-3]

### Example 68

### Isobutyric acid 1-{(1 S,3S)-1-((S)-1-hydroxy-2-piperidin-1-yl-ethyl)-3-[4-methoxy-3-(3-methoxy-propoxy)-benzyl]-4-methyl-pentylcarbamoyloxy}-ethyl ester (diastereomeric mixture)

To a stirred solution of 1 mmol of (1S,3S)-1-((S)-1-hydroxy-2-piperidin-1-yl-ethyl)-3-[4-methoxy-3-(3-methoxy-propoxy)-benzyl]-4-methyl-pentyll-carbamic acid 1-chloro-ethyl ester (diastereomeric mixture) in 30 ml of N,N-dimethylformamide is added 5 mmol caesium carbonate, followed by 2 mmol of isobutyric acid [79-31-2]. The reaction mixture is stirred for 12 hours at room temperature. The reaction mixture is diluted with water and extracted with tert-butyl methyl ether (4X). The combined organic phases are washed with brine, dried over sodium sulfate and the solvent is concentrated under reduced pressure. The residue is purified by flash chromatography (SiO₂ 60F) to afford the title compound, which is identified based on its Rf value.

The starting materials are prepared as follows:
a) {(1S,3S)-1-((S)-1-Hydroxy-2-piperidin-1-yl-ethyl)-3-[4-methoxy-3-(3-methoxy-propoxy)-benzyl]-4-methyl-pentyl}-carbamic acid 1-chloro-ethyl ester (diastereomeric mixture)
   To a stirred solution of 1 mmol of (2S,3S,5S)-3-amino-5-[4-methoxy-3-(3-methoxy-propoxy)-benzyl]-6-methyl-1-piperidin-1-yl-heptan-2-ol [861922-24-9] in 20 ml of dichloromethane is added 3 mmol triethylamine. The solution is cooled to 0°C and 1.1 mmol of 1-chloroethyl chloroformate [50893-53-3] is added. The reaction mixture is stirred for 5 minutes at 0°C, then for 3 hours at room temperature. The reaction mixture is diluted with dichloromethane and washed with 1M hydrochloric acid. The aqueous phase is re-extracted with dichloromethane (1X). The combined organic phases are washed with 1M aqueous sodium bicarbonate solution, dried over sodium sulfate and the solvent is concentrated under reduced pressure. The residue is purified by flash chromatography (SiO₂ 60F) to afford the title compound, which is identified based on its Rf value.

According to the procedure described in example 68, the following compounds are prepared in analogous manner:
- 74: Isobutyric acid 1-((1S,3S)-1-((S)-1-hydroxy-2-piperidin-1-yl-ethyl)-3-{[2-(3-methoxy-propoxy)-benzoylamino]-methyl}-4-methyl-pentylcarbamoyloxy)-ethyl ester (diastereomeric mixture)
starting from N-((2S,4S,5S)-4-amino-5-hydroxy-2-isopropyl-6-piperidin-1-yl-hexyl)-2-(3-methoxy-propoxy)-benzamide [861772-91-0]
- 80: Isobutyric acid 1-[(S)-1-((S)-1-hydroxy-2-piperidin-1-yl-ethyl)-5-(3-methoxycarbonylamino-3,4-dihydro-2H-quinolin-1-yl)-3,3-dimethyl-5-oxo-pentylcarbamoyloxy]-ethyl ester (diastereomeric mixture)
starting from [1-((5S,6S)-5-amino-6-hydroxy-3,3-dimethyl-7-piperidin-1-yl-heptanoyl)-1,2,3,4-tetrahydro-quinolin-3-yl]-carbamic acid methyl ester [861444-61-3]

### Example 69

### trans-(S)-2-Amino-3-methyl-butyric acid 4-(1-{(2S,3S,5S)-3-amino-2-hydroxy-5-[4-methoxy-3-(3-methoxy-propoxy)-benzyl]-6-methyl-heptylcarbamoyl}-1-methyl-ethyl)-cyclohexyl ester, bis(trifluoroacetate) (diastereomeric mixture)

According to general procedure Q, trans-(S)-2-tert-butoxycarbonylamino-3-methyl-butyric acid 4-(1-{(2S,3S,5S)-3-tert-butoxycarbonylamino-2-hydroxy-5-[4-methoxy-3-(3-methoxy-propoxy)-benzyl]-6-methyl-heptylcarbamoyl}-1-methyl-ethyl)-cyclohexyl ester (diastereomeric mixture) is used to afford the title compound, which is identified based on its Rf value.

The starting materials are prepared as follows:
a) trans-(S)-2-tert-Butoxycarbonylamino-3-methyl-butyric acid 4-(1-{(2S,3S,5S)-3-tert-butoxycarbonylamino-2-hydroxy-5-[4-methoxy-3-(3-methoxy-propoxy)-benzyl]-6-methyl-heptylcarbamoyl}-1-methyl-ethyl)-cyclohexyl ester (diastereomeric mixture)
   According to general procedure D, {(1S,3S)-1-((S)-2-amino-1-hydroxy-ethyl)-3-[4-methoxy-3-(3-methoxy-propoxy)-benzyl]-4-methyl-pentyl}-carbamic acid tert-butyl ester [861901-11-3] and trans-(S)-2-tert-butoxycarbonylamino-3-methyl-butyric acid 4-(1-carboxy-1-methyl-ethyl)-cyclohexyl ester (diastereomeric mixture) are used to afford the title compound, which is identified based on its Rf value.
b) trans-(S)-2-tert-Butoxycarbonylamino-3-methyl-butyric acid 4-(1-carboxy-1-methylethyl)-cyclohexyl ester (diastereomeric mixture)
   According to general procedure I, trans-(S)-2-tert-butoxycarbonylamino-3-methyl-butyric acid 4-(1-methoxycarbonyl-1-methyl-ethyl)-cyclohexyl ester (diastereomeric mixture) is used to afford the title compound, which is identified based on its Rf value.
c) trans-(S)-2-tert-Butoxycarbonylamino-3-methyl-butyric acid 4-(1-methoxycarbonyl-1-methyl-ethyl)-cyclohexyl ester (diastereomeric mixture)
   According to general procedure D, trans-2-(4-hydroxy-cyclohexyl)-2-methyl-propionic acid methyl ester [861444-86-2] and (S)-2-tert-butoxycarbonylamino-3-methyl-butyric acid [13734-41-3] are used to afford the title compound, which is identified based on its Rf value.

According to the procedure described in example 68, the following compounds are prepared in analogous manner:
- 75: (S)-2-Amino-3-methyl-butyric acid 4-[1-((2S,3S,5S)-3-amino-2-hydroxy-5-{[2-(3-methoxy-propoxy)-benzoylamino]-methyl}-6-methyl-heptylcarbamoyl)-1-methyl-ethyl]-cyclohexyl ester, bis(trifluoroacetate) (diastereomeric mixture)
starting from ((1S,3S)-1-((S)-2-amino-1-hydroxy-ethyl)-3-{[2-(3-methoxy-propoxy)-benzoylamino]-methyl}-4-methyl-pentyl)-carbamic acid tert-butyl ester [861451-17-4]
- 81: (S)-2-Amino-3-methyl-butyric acid 4-{1-[(2S,3S)-3-amino-2-hydroxy-7-(3-methoxycarbonylamino-3,4-dihydro-2H-quinolin-1-yl)-5,5-dimethyl-7-oxo-heptylcarbamoyl]-1-methyl-ethyl}-cyclohexyl ester, bis(trifluoroacetate) (diastereomeric mixture)
starting from [1-((5S,6S)-7-amino-5-tert-butoxycarbonylamino-6-hydroxy-3,3-dimethyl-heptanoyl)-1,2,3,4-tetrahydro-quinolin-3-yl]-carbamic acid methyl ester [861444-82-8]

## Claims

1. Process for enhancing the membrane transport of compounds of the general formula (I) 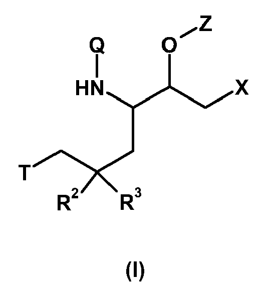 and the salts thereof, preferably the pharmaceutically acceptable salts thereof; where R¹ is aryl or a nitrogen-containing heterocyclyl, especially benzoimidazolyl, benzooxazolyl, benzothiazolyl, dihydro-2H-benzo[1,4]oxazinyl, dihydro-3H-benzo[1,4]oxazinyl, dihydro-2H-benzo[1,4]thiazinyl, dihydro-1H-pyrido[2,3-b][1,4]oxazinyl, imidazo[1,2-a]pyridyl, imidazo[1,5-a]pyridyl, indazolyl, indolyl, isoquinolyl, [1,5]naphthyridyl, phenyl, phthalazinyl, pyridyl, pyrimidinyl, 1H-pyrrolo[2,3-b]pyridyl, 1H-pyrrolo[2,3-c]pyridyl, 1H-pyrrolo[3,2-b]pyridyl, quinazolinyl, quinolyl, quinoxalinyl, tetrahydroimidazo[1,2-a]pyridyl, tetrahydroimidazo[1,5-a]pyridyl, tetrahydroisoquinolyl, tetrahydroquinolyl, tetrahydroquinoxalinyl, [1,2,3]triazolo[1,5-a]pyridyl or [1,2,4]triazolo[4,3-a]pyridyl, each of which is substituted by 1-4 acyl-C₁₋₈-alkoxy-C₁₋₈-alkoxy, acyl-C₁₋₈-alkoxy-C₁₋₈-alkyl, (N-acyl)-C₁₋₈-alkoxy-C₁₋₈-alkylamino, C₁₋₈-alkanoyl, C₁₋₈-alkoxy, C₁₋₈-alkoxy-C₁₋₈-alkanoyl, C₁₋₈-alkoxy-C₁₋₈-alkoxy, C₁₋₈-alkoxy-C₁₋₈-alkoxy-C₁₋₈-alkyl, C₁₋₈-alkoxy-C₁₋₈-alkyl, (N-C₁₋₈-alkoxy)-C₁₋₈-alkylaminocarbonyl-C₁₋₈-alkoxy, (N-C₁₋₈-alkoxy)-C₁₋₈-alkylaminocarbonyl-C₁₋₈-alkyl, C₁₋₈-alkoxy-C₁₋₈-alkyl-carbamoyl, C₁₋₈-alkoxy-C₁₋₈-alkyl-carbonylamino, C₁₋₈-alkoxy-C₁₋₈-alkyl-heterocydyl, C₁₋₈-alkoxy-carbonyl, C₁₋₈-alkoxy-carbonyl-C₁₋₈-alkoxy, C₁₋₈-alkoxycarbonyl-C₁₋₈-alkyl, C₁₋₈-alkoxycarbonylamino, C₁₋₈-alkoxy-carbonylamino-C₂₋₈-alkoxy, C₁₋₈-alkoxycarbonylamino-C₁₋₈-alkyl, C₁₋₈-alkyl, (N-C₁₋₈-alkyl)-C₁₋₈-alkoxy-C₁₋₈-alkyl-carbamoyl, (N-C₁₋₈-alkyl)-C₁₋₈-alkoxy-C₁₋₈-alkylcarbonylamino, (N-C₁₋₈-alkyl)-C₁₋₈-alkoxy-carbonylamino, (N-C₁₋₈-alkyl)-C₁₋₈-alkylcarbonylamino-C₂₋₈-alkoxy, (N-C₁₋₈-alkyl)-C₁₋₈-alkylcarbonylamino-C₁₋₈-alkyl, (N-C₁₋₈-alkyl)-C₁₋₈-alkylsulfonylamino-C₂₋₈-alkoxy, (N-C₁₋₈-alkyl)-C₁₋₈-alkylsulfonylamino-C₁₋₈-alkyl, C₁₋₈-alkyl-amidinyl, C₁₋₈-alkylamino-C₂₋₈-alkoxy, di-C₁₋₈-alkylamino-C₂₋₈-alkoxy, C₁₋₈-alkylamino-C₁₋₈-alkyl, di-C₁₋₈-alkylamino-C₁₋₈-alkyl, C₁₋₈-alkylaminocarbonyl-C₁₋₈-alkoxy, di-C₁₋₈-alkylaminocarbonyl-C₁₋₈-alkoxy, C₁₋₈-alkylamino-carbonyl-C₁₋₈-alkoxy-C₁₋₈-alkyl, C₁₋₈-alkylaminocarbonyl-C₁₋₈-alkyl, di-C₁₋₈-alkylaminocarbonyl-C₁₋₈-alkyl, C₁₋₈-alkylaminocarbonylamino-C₂₋₈-alkoxy, C₁₋₈-alkylaminocarbonylamino-C₁₋₈-alkyl, C₁₋₈-alkylcarbonylamino, C₁₋₈-alkylcarbonylamino-C₂₋₈-alkoxy, C₁₋₈-alkylcarbonylamino-C₁₋₈-alkyl, C₁₋₈-alkylcarbonyloxy-C₂₋₈-alkoxy, C₁₋₈-alkylcarbonyloxy-C₁₋₈-alkyl, C₁₋₈-alkylsulfonyl, C₁₋₈-alkylsulfonyl-C₁₋₈-alkoxy, C₁₋₈-alkylsulfonyl-C₁₋₈-alkyl, C₁₋₈-alkylsulfonylamino-C₂₋₈-alkoxy, C₁₋₈-alkylsulfonylamino-C₁₋₈-alkyl, optionally N-mono- oder N,N-di-C₁₋₈-alkylated amino, aryl-C₀₋₈-alkoxy, aryl-C₀₋₈-alkyl, optionally N-mono- oder N,N-di-C₁₋₈-alkylated carbamoyl-C₀₋₈-alkoxy, carboxy-C₁₋₈-alkoxy, carboxy-C₁₋₈-alkoxy-C₁₋₈-alkyl, carboxy-C₁₋₈-alkyl, C₁₋₈-alkyl, cyano-C₂₋₈-alkoxy, cyano-C₁₋₈-alkyl, C₃₋₈-cycloalkyl-C₁₋₈-alkoxy, C₃₋₈-cycloalkyl-carbamoyl-C₀₋₈-alkyl, C₃₋₈-cycloalkylcarbonylamino-C₂₋₈-alkoxy, C₃₋₈-cycloalkylcarbonylamino-C₁₋₈-alkyl, O,N-dimethylhydroxylamino-C₁₋₈-alkyl, halogen, halogen-C₁₋₈-alkoxy, halogen-C₁₋₈-alkyl, heterocydyl-C₀₋₈-alkoxy, heterocydyl-C₀₋₈-alkyl, heterocyclyl-carbonyl, hydroxy-C₂₋₈-alkoxy-C₁₋₈-alkoxy, hydroxy-C₂₋₈-alkoxy-C₁₋₈-alkyl, hydroxy-C₁₋₈-alkyl, O-methyloximyl-C₁₋₈-alkyl, oxide or oxo;
R² und R³ are, independently from each other, hydrogen or C₁₋₈-alkyl or, together with the carbon atom they are linked to, are C₃₋₈-cydoalkyl;
R⁴ is C₁₋₈-alkyl, C₁₋₈-hydroxyalkyl, C₁₋₈-alkoxy-C₁₋₈-alkyl, C₁₋₈-alkanoyloxy-C₁₋₈-alkyl, C₁₋₈-aminoalkyl, C₁₋₈-alkylamino-C₁₋₈-alkyl, C₁₋₈-dialkylamino-C₁₋₈-alkyl, C₁₋₈-alkanoylamido-C₁₋₈-alkyl, HO(O)C-C₁₋₈-alkyl, C₁₋₈-alkyl-O-(O)C-C₁₋₈-alkyl, H₂N-C(O)-C₁₋₈-alkyl, C₁₋₈-alkyl-HN-C(O)-C₁₋₈-alkyl, (C₁₋₈-alkyl)₂N-C(O)-C₁₋₈-alkyl, C₂₋₈-alkenyl, C₂₋₈-alkynyl, cyano-C₁₋₈-alkyl, halo-C₁₋₈-alkyl, optionally substituted aryl-C₀₋₈-alkyl, optionally substituted C₃₋₈-cydoalkyl-C₀₋₈-alkyl or optionally substituted heterocyclyl-C₀₋₈-alkyl
whereby,
each of the residues, mentioned above for R⁴, bearing a hydroxy-group are optionally substituted at that hydroxy-group by a radical (A) whereby an ester bond is formed; ;
R⁵ is hydrogen or C₁₋₈-alkyl;
R⁶ is optionally carboxy- or hydroxy-substituted C₁₋₈-alkyl or optionally carboxy- or hydroxy-substituted aryl-C₀₋₈-alkyl;
R⁷ is C₁₋₈-alkyl;
R⁸ and R⁹, together with the nitrogen atom to which they are bonded, are a 5-7-membered ring;
(A) represents a mono- or di-peptidic residue of one or two of the 20 natural amino acids, said residue being formally obtained from the mono- or di-peptide by elimination of the OH-group from the C-terminal end;
Q is hydrogen or represents a radical (A) whereby an amide bond is formed or a group of the formula -C(O)OCHR⁶OC(O)R⁷;
T is R¹, R¹-C(O)- or R¹-C(O)-NR⁵-;
X is -NR⁵-C(O)R⁴, -Alk-C(O)-NR⁵R⁴ or -NR⁸R⁹, where Alk represents C₁₋₈-alkylene; and
Z is hydrogen or represents a radical (A) whereby an ester bond is formed;
**characterized in that**
(i) a radical (A) is present in at least one of R⁴, Q or Z; or else
(ii) at least Q is a group of the formula -C(O)OCHR⁶OC(O)R⁷.

2. Compound of the general formula (I) 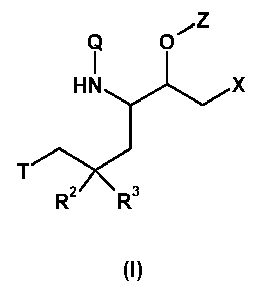 and the salts thereof, preferably the pharmaceutically acceptable salts thereof; where
A) X is -NR⁵-C(O)R⁴, -Alk-C(O)-NR⁵R⁴ or -NR⁸R⁹, where Alk represents C₁₋₈-alkylene; and
T is R¹, R¹-C(O)- or R¹-C(O)-NR⁵-; and
R¹ is a substituted nitrogen-containing heterocyclyl; or
B) X is -NR⁵-C(O)R⁴or-NR⁸R⁹, where Alk represents C₁₋₈-alkylene; and
T is R¹, R¹-C(O)- or R¹-C(O)-NR⁵-; and
R¹ is substituted aryl; or
C) X is -Alk-C(O)-NR⁵R⁴ , where Alk represents C₁₋₈-alkylene and R⁴ is tetrahydropyranyl-C₀-C₄-alkyl; and
T is R¹; and
R¹ is substituted aryl;
R² und R³ are, independently from each other, hydrogen or C₁₋₈-alkyl or, together with the carbon atom they are linked to, are C₃₋₈-cycloalkyl;
R⁴ is C₁₋₈-alkyl, C₁₋₈-hydroxyalkyl, C₁₋₈-alkoxy-C₁₋₈-alkyl, C₁₋₈-alkanoyloxy-C₁₋₈-alkyl, C₁₋₈-aminoalkyl, C₁₋₈-alkylamino-C₁₋₈-alkyl, C₁₋₈-dialkylamino-C₁₋₈-alkyl, C₁₋₈-alkanoylamido-C₁₋₈-alkyl, HO(O)C-C₁₋₈-alkyl, C₁₋₈-alkyl-O-(O)C-C₁₋₈-alkyl, H₂N-C(O)-C₁₋₈-alkyl, C₁₋₈-alkyl-HN-C(O)-C₁₋₈-alkyl, (C₁₋₈-alkyl)₂N-C(O)-C₁₋₈-alkyl, C₂₋₈-alkenyl, C₂₋₈-alkynyl, cyano-C₁₋₈-alkyl, halo-C₁₋₈-alkyl, optionally substituted aryl-C₀₋₈-alkyl, optionally substituted C₃₋₈-cycloalkyl-C₀₋₈-alkyl or optionally substituted heterocyclyl-C₀₋₈-alkyl
whereby,
each of the residues, mentioned above for R⁴, bearing a hydroxy-group are optionally substituted at that hydroxy-group by a radical (A) whereby an ester bond is formed;
R⁵ is hydrogen or C₁₋₈-alkyl;
R⁶ is optionally carboxy- or hydroxy-substituted C₁₋₈-alkyl or optionally carboxy- or hydroxy-substituted aryl-C₀₋₈-alkyl;
R⁷ is C₁₋₈-alkyl;
R⁸ and R⁹, together with the nitrogen atom to which they are bonded, are a 5-7-membered ring;
(A) represents a mono- or di-peptidic residue of one or two of the 20 natural amino acids, said residue being formally obtained from the mono- or di-peptide by elimination of the OH-group from the C-terminal end;
Q is hydrogen or represents a radical (A) whereby an amide bond is formed or a group of the formula -C(O)OCHR⁶OC(O)R⁷;
Z is hydrogen or represents a radical (A) whereby an ester bond is formed; and
(i) a radical (A) is present in at least one of R⁴, Q or Z; or else
(ii) at least Q is a group of the formula -C(O)OCHR⁶OC(O)R⁷.

3. A compound according to claim 2 of the general formula (IA) 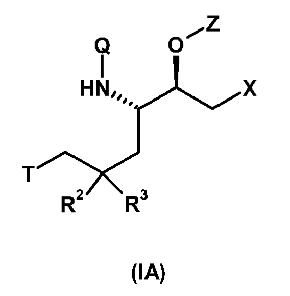 and the salts thereof, preferably the pharmaceutically acceptable salts thereof; in which R², R³, Q, T, X and Z have the meaning according to claim 2 for the compounds of the formula (I).

4. A compound according to claim 2 or 3, wherein
R⁴ is optionally substituted aryl-C₀₋₈-alkyl, optionally substituted C₃₋₈-cydoalkyl-C₀₋₈-alkyl or optionally substituted heterocyclyl-C₀₋C₆-alkyl,
whereby,
each of the residues, mentioned above for R⁴, bearing a hydroxy-group is optionally substituted at that hydroxy-group by a radical (A).

5. A compound according to claim 2 or 3, wherein
A) X is -NR⁵-C(O)R⁴, -Alk-C(O)-NR⁵R⁴ or -NR⁸R⁹, where Alk represents C₁₋₈-alkylene; and
T is R¹, R¹-C(O)- or R¹-C(O)-NR⁵-; and
R¹ is a substituted benzoimidazolyl, 3,4-dihydro-2H-benzo[1,4]oxazinyl, indazolyl, indolyl or pyridyl; or
B) X is -NR⁵-C(O)R⁴ or -NR⁸R⁹, where Alk represents C₁₋₈-alkylene; and
T is R¹, R¹-C(O)- or R¹-C(O)-NR⁵-; and
R¹ is substituted phenyl; or
C) X is -Alk-C(O)-NR⁵R⁴, where Alk represents C₁₋₈-alkylene and R⁴ is tetrahydropyranyl-C₀-C₄-alkyl; and
T is R¹; and
R¹ is substituted phenyl;
R² und R³ are, independently from each other, hydrogen or C₁₋₆-alkyl or, together with the carbon atom they are linked to, are C₃₋₈-cycloalkyl;
R⁴ is optionally substituted aryl-C₀₋₈-alkyl, optionally substituted C₃₋₈-cycloalkyl-C₀₋₈-alkyl or optionally substituted heterocyclyl-C₀₋C₆-alkyl,
whereby,
each of the residues, mentioned above for R⁴, bearing a hydroxy-group is optionally substituted at that hydroxy-group by a radical (A);
R⁵ is hydrogen or C₁₋₈-alkyl;
R⁶ is optionally carboxy- or hydroxy-substituted C₁₋₈-alkyl or aryl-C₀₋₈-alkyl;
R⁷ is C₁₋₈-alkyl;
R⁸ and R⁹, together with the nitrogen atom to which they are bonded, are a 5-7-membered ring;
(A) represents a mono- or di-peptidic residue of one or two of the 20 natural amino acids, said residue being formally obtained from the mono- or di-peptide by elimination of the OH-group from the C-terminal end;
Q is hydrogen or represents a radical (A) or a group of the formula -C(O)OCHR ⁶OC(O)R⁸;
Z is hydrogen or represents a radical (A);
and whereby
(i) a radical (A) is present in at least one of R⁴, Q or Z; or else
(ii) at least Q is a group of the formula -C(O)OCHR⁶OC(O)R⁷.

6. Use of a compound according to any one of claims 2 to 5 for the preparation of a medication, in particular a medication for the treatment or prevention of high blood pressure, heart failure, glaucoma, myocardial infarction, renal failure, restenoses and stroke.

7. A pharmaceutical composition comprising a compound according to any one of claims 2 to 5 and optionally one or more agents having cardiovascular activity.
